(19) Europäisches Patentamt

European Patent Office

Office européen des brevets

(11) **EP 1 161 940 B1**

(12) **EUROPEAN PATENT SPECIFICATION**

(45) Date of publication and mention
of the grant of the patent:
**08.06.2005 Bulletin 2005/23**

(51) Int Cl.⁷: **A61K 9/20**, A61K 47/26

(21) Application number: **00903982.7**

(86) International application number:
**PCT/JP2000/000870**

(22) Date of filing: **16.02.2000**

(87) International publication number:
**WO 2000/048575 (24.08.2000 Gazette 2000/34)**

(54) **TABLETS AND PROCESS FOR PRODUCING TABLETS**

TABLETTEN UND VERFAHREN ZUR TABLETTENHERSTELLUNG

COMPRIMES ET PROCEDE DE PRODUCTION DE COMPRIMES

(84) Designated Contracting States:
**AT BE CH CY DE DK ES FI FR GB GR IE IT LI LU MC NL PT SE**
Designated Extension States:
**AL LT LV MK RO SI**

(30) Priority: **17.02.1999 JP 3929499**

(43) Date of publication of application:
**12.12.2001 Bulletin 2001/50**

(73) Proprietor: **KYOWA HAKKO KOGYO CO., LTD.**
**Chiyoda-ku, Tokyo 100-8185 (JP)**

(72) Inventors:
 • **WATANABE, Yasushi**
  **Sunto-gun, Shizuoka 411-8731 (JP)**
 • **MORIMOTO, Kiyoshi**
  **Sunto-gun, Shizuoka 411-8731 (JP)**

 • **IWASE, Yuji**
  **Sunto-gun, Shizuoka 411-8731 (JP)**
 • **HIRUTA, Satoru**
  **Sunto-gun, Shizuoka 411-8731 (JP)**

(74) Representative: **VOSSIUS & PARTNER**
**Siebertstrasse 4**
**81675 München (DE)**

(56) References cited:
**EP-A1- 0 745 382     EP-A2- 0 553 777**
**JP-A- 11 012 161     JP-A- 11 263 723**
**US-A- 5 660 860**

 • **BAUER K.H. ET AL.: "Pharmazeutische Technologie" 1991 , GEORG THIEME VERLAG , STUTTGART XP002204249 * page 300; table 14.1 ***

EP 1 161 940 B1

## Description

Technical Field

The present invention relates to a tablet and a tablet production method, and relates particularly to an intrabuccally rapidly disintegrable tablet and to a method of manufacturing such tablet.

Background Art

There are various types of tablets used in the medical and clinical field. However, few tablets fulfill certain patients needs in terms of dosage. In particular, there is a need for tablets that can be easily dosed for elderly people and children having difficulties swallowing.

An intrabuccally rapidly disintegrable tablet is characterized by being disintegrated rapidly in the mouth and by being capable of being advantageously taken without water by elderly person or children having difficulties swallowing.

Intraorally rapidly disintegrable tablet have been produced by moistening a mass including an active substance with ethanol and/or water and by drying it after molding (JP-A-2-32014).

JP-A-5-271054 discloses a method of producing an intrabuccally rapidly disintegrable tablet wherein a powdered mixture including an active substance, a saccharide, and an amount of water sufficient for wetting the particle surface of the saccharide is compressed to make tablets before drying. WO95/20380 discloses a tablet comprising a saccharide such as maltose used as binder compressed with a granulated material comprising at least a principal agent and a saccharide with low moldability such as lactose to produce a tablet.

However the methods of manufacturing tablets described in JP-A-2-32014 and in JP-A-5-271054 require a special manufacturing technology. This creates a problem of low productivity. Further in the tablet described in JP-A-5-271054, the particles are bound together by saccharide. The bonds between the particles may be weak so that the tablets can easily be chipped during storage and transportation.

The oral formulation disclosed in WO95/20380, is rapidly disintegrated in the mouth but its moldability is not sufficient and its tabletting difficult. Because particles are bound together by saccharide, the bonds are weak and the tablets may easily be chipped during storage and transportation.

Disclosure of the Invention

The present invention has been proposed to solve the above-mentioned problems. An object of the invention is to provide a tablet which is superior in disintegration and moldability in case of tabletting, has adequate hardness after compression, has superior characteristics decreasing the risks of chipping tablets during storage and transportation, the case being, and that can be produced by conventional production methods, and to provide its production method.

The present invention relates to the following.

(1) The present invention relates to a tablet obtainable by binding a mixture of a principal agent, a saccharide with high wettability against water, and a disintegrant with a binder including a saccharide with high wettability against water and by compressing the granulated material wherein said binder is a water-soluble polymer and said disintegrant is selected from the group consisting of crosspovidone, crosscarmellose sodium, and low substituted hydrodxypropylcellulose.

The term "principal agent" used in the present invention generally relates to the basis in a tablet having a therapeutic activity expected by prescription and has the same meaning as an active component, an effective ingredient, an active substance, or an active ingredient. In pharmaceutical compositions, the term relates to a powder or a granule constituted of a principal agent, or to a powder or a granule mainly including a principal agent when a tablet is produced by compressing a powdered or granular material.

The composition amount of the principal agent is preferably less than or equal to 50 volumes / volume percentage (v/v%) and is more preferably below 20 volumes / volume percentage (v/v%), although it depends on its physical, chemical and pharmacological property.

When the "principal agent" possesses a peculiar flavor and odor when it may interact with an other component included in a tablet, when the principal agent is transported to a target tissue while being protected , when the release speed needs to be adjusted, or when the principal agent needs to be protected from an environmental factor such as oxygen, water, and light, it is preferable that the powder or granule consisting of a principal agent, or the powder or granule of which the main ingredient is a principal agent be coated with a coating agent.

The methods of coating a powder or granule consisting of a principal agent, or a powder or granule of which the main ingredient is a principal agent are not limited. Several methods such as Pan coating, fluid-bed coating or an air-suspension coating may be used for instance.

A powder or granule comprising a principal agent, or a powder or granule of which the main ingredient is a principal agent may be obtained by grinding a solid dispersing material.

Further, when the release speed needs to be adjusted, a powder or granule comprised of a principal agent, or a powder or granule of which the main ingredient is a principal agent may be a powder or granule obtained by grinding a matrix type pharmaceutical compound in which a principal agent is dissolved or dispersed homogeneously or non-homogeneously in a high molecular web formation.

When the term "powder" is used in this specification, it refers to an aggregation of particles of which diameter (particle size) is greater than or equal to 0.1 µm and less than or equal to 100 µm. When the term "granule" is used, it refers to an aggregation of particles of which diameter (particle size) is greater than or equal to 100 µm. When the term "granulated material" is used, it means an aggregation in which one particle is comprised of an aggregation of particles.

The term "a saccharide with high wettability against water" means a saccharide which is superior in wettablity in water and of which viscosity increase is less when a fixed amount of a saccharide is dissolved in a fixed amount of water.

More specifically, "a saccharide with high wettability against water" means a saccharide which satisfies either kinetic viscosity of a sample solution having density of 1.0g/100ml is less than or equal to 0.92 cm stoke (cSt), or a solubility for water of 25°C is less than or equal to 18 weight % when measured with the Ubbelohde viscosimeter according to a viscosity measuring method defined by the General Test Procedures of Japanese Pharmacopoeia, 13$^{th}$ edition.

It is well known that the wettability of a powder layer follows a disintegration model of Washburn and is shown like in the following experimental formula;

$$T = 2\eta L/r \, \nu \cos\theta$$

T : required time for a fixed amount of water to be permeated in a powder layer
: viscosity of permeating water
L: apparent capillary length
r: apparent capillary radius
v: surface tension of permeating water
θ: angle of contact

When the powder layer is a tablet, apparent capillary length L and apparent capillary radius r are decided by the density of the tablet. The volume is constant in case of a tablet, so L/r in the above experimental formula can be read as a free volume.

Considering that a saccharide has a basically hydrophilic property, the angle of contact θ does not become a dominant factor (governing factor) affecting the time T required for permeating a fixed amount of water into a powder layer (tablet), so that the angle of contact θ can be removed from a wettable factor of a powder layer.

Therefore, a viscosity of permeating water η and a surface tension of permeating water ν become a dominant factor (governing factor) affecting the time T required for permeating a fixed amount of water into the powder layer (tablet).

In the mouth, saliva permeates in the tablet and affects the wettablity of the tablet. When saliva is considered as water, the surface tension of water is 0.85 cm stoke (cSt) (square millimeter/second (mm$^2$/s)). It can be considered that the surface tension of solution in which a saccharide is dissolved in water does not change so much comparing with that of water.

Accordingly, it can be said that a dominant factor (governing factor) affecting the time T required for permeating into a powder layer is the viscosity of permeating water η.

Therefore, it is preferable that a solution with low viscosity be used in order to shorten the time T required for a fixed amount of water to be permeated in a powder layer (tablet) when a saccharide is dissolved in water.

A saccharide having low solution viscosity was examined by dissolving 0.5g of various kinds of saccharide in 50ml of water.

As a result, powder of trehalose (kinetic viscosity: 0.891cSt), mannitol (kinetic viscosity : 0.896cSt), maltose (kinetic viscosity : 0.896cSt), sorbitol (kinetic viscosity : 0.897cSt), lactose (kinetic viscosity : 0.897cSt), multitol (kinetic viscosity : 0.904cSt), xylitol (kinetic viscosity : 0.904cSt), sucrose (kinetic viscosity : 0.912cSt), erythritol (kinetic viscosity : 0.912cSt), and glucose (kinetic viscosity : 0.895cSt) was granulated while spraying a binder solution by means of spray means according to a fluid-bed granulation method and thus granulated material was compressed and molded as a tablet. It was found by experiments that a tablet using such a saccharide was rapidly disintegrated by saliva in the mouth.

It was further found that a tablet produced by compressing granulated material comprising a binder solution wherein these saccharides were dissolved or a binder solution in which a surface active agent other than' these saccharides was dissolved, was rapidly disintegrated by the saliva in the mouth as compared to what occurred with a tablet produced by compressing granulated material comprising a conventional binder solution.

Mannitol is listed as a saccharide which satisfies the condition that a solubility in water of 25°C is less than or equals to 18 weight %.

18.19g of mannitol is dissolved in 100ml of water (25°C) and its solubility is about 18 weight/volume% (w/v%) so that mannitol is listed as a group with the lowest solubility.

A disintegrant is used as the agent of the tablet permeated by the moisture of the saliva thereby helping it being disintegrated and dispersed into particle sizes.

Many types of disintegrant can be used according to the present invention and they are not specifically limited.

Crosspovidone, cross sodium carboxymethyl cellulose, low substituted hydroxypropylcellulose, sodium carboxymethylsatarch, sodium alginate, carmellose, sodium carboxymethylcellulose, calcium carboxymethylcellulose, agar powder, gelatin, shellac, crystalline cellulose, calcium carbonate, sodium bicarbonate, starch such as corn starch and potato starch, sodium starch glycolate, tragacanth, methylcellulose (MC), pregelatinized starch (HPS), bentonite, sodium lauryl sulfate, calcium phosphate, povidone are used as disintegrant.

For rapid release tablets, crosspovidone, cross sodium carboxymethyl cellulose, low substituted hydroxypropylcellulose, and sodium carboxymethylsatarch are preferable.

Although several kinds of binder can be used, water-soluble polymer is preferably used for rapid disintegration of a tablet in the mouth. For example, hydroxypropylcellulose, polyvinylpyrrolidone, hydroxypropylmethylcellulose, partially saponified polyvinyl alcohol, methylcellulose (HPMC), pullulane, polyvinyl alcohol (PVA), and hydroxypropylcellulose (HPC) can be used for this purpose.

The particle size of a saccharide with high wettability against water and the particle size of a disintegrant are preferably greater than or equal to 10 $\mu$m to less than or equal to 500 $\mu$m, more preferably greater than or equal to 20 $\mu$m to less than or equal to 300 $\mu$m, and even more preferably greater than or equal to 20 $\mu$m to less than or equal to 200 $\mu$m if considering the moldability and disintegration of a tablet in the mouth.

Granulated material obtained by binding a mixture including at least a principal agent, a saccharide with high wettability against water, and a disintegrant by means of a binder including a saccharide with high wettability against water may be compressed to produce a tablet without adding other components. Or a lubricant, a corrigent, a foaming agent, a diluting agent, a disintegrant auxiliaries, an aromatic, a solubilizing agent, a coloring agent, a fluidizing agent and other adjuvants may be added and thereafter the granulated material may be compressed to produce a tablet.

The amount of adjuvant used differs according to the expected disintegration time of the tablet and is not particularly limited. If a lubricant is added, the amount of lubricant is preferably greater than or equal to 0.01 weight% and less than or equal to 10 weight%, preferably greater than or equal to 0.1 weight% and less than or equal to 5 weight%, and more preferably 0.5 weight% and less than or equal to 3 weight% for the weight of one produced tablet.

The reason of such a limitation is that the rapid disintegration which is an original function of the tablet of the present invention may be spoiled and the disintegration time becomes slow when the amount of added lubricant exceeds the above range.

On the other hand, when the amount of added lubricant is below the above-mentioned range, molding material may attach on punches and dies of the tabletting machine so that the punches and dies may be ground and tabletting problems such as sticking, capping, laminating, and binding may occur, thereby decreasing productivity.

Lubricants that may be used according to the present invention are not limited and several kinds of lubricant may be used. Stearic acid, salt stearate (Al, K, Na, Ca, Mg), stearyl alcohl, sucrose esters of fatty acid, talc, carnauba wax, light anhydrous silicic acid, magnesium silicate, synthetic aluminum silicate, hardened oil, white beeswax, titanium oxide, talc, corn starch, microcrystalline cellulose, macrogol 4000, macrogol 6000, isopropyl myristate, magnesium lauryl sulfate, dibasic calcium phosphate, and wax may be used for instance.

The amount of adjuvant to be used among the above-mentioned other than a lubricant varies according to the physical and chemical property of the principal agent, the object of the tablet, and the size of the tablet so that it is difficult to determine generally. However, the amount of such adjuvant is preferably minimum. The use of a large amount of such adjuvant with the granulated material defeats the granulated material's purpose and other components' function becomes dominant.

The types of corrigent that may be used herein are not limited and several kinds of sweetening agent and several kinds of flavoring agent may be used. Sweetening agents include, for example, aspartame, saccharin, saccharin sodium, glycyrrhizin (glycyrrhizic acid), trisodium glycyrrhizinate, disodium glycyrrhizinate, powdered sweet hydrangea leaf, glycyrrhiza (powder, extract), syrup, sucrose, honey, and D-mannitol. The types of flavoring agents that may be used herein include but are not limited to cacao butter, citric acid, monosodium glutaminate, tartaric acid, and ginger other than several kinds of aromatic agent and several kinds of sweetening agent.

The types of forming agent that may be used herein are not limited and several kinds can be used, for example, sodium bicarbonate, sodium carbonate, and calcium carbonate.

The types of diluting agents that may be used herein are not limited and several kinds can be used, for example, lactose, corn starch, and crystalline cellulose.

The types of disintegrants that may be used herein are not limited and several kinds can be used, for example, solubilizing agent, emulsifier, suspension and dispersant.

The types of solubilizing agents that may be used herein are not limited and several kinds can be used, for example, sodium oleate, polyoxil stearate, propylene carbonate, polyoxyethylene lauryl ether, polysorbate 80, isopropyl myristate, and lauromacrogol.

The types of emulsifiers that may be used herein are not limited and several kinds can be used, for example, acacia gum, cholesterol, sodium carboxymethylcellulose, polyoxil stearate 40, sorbitan sesquioleate, methylcellulose (HPMC), BEGUM (aluminum magnesium silicate, "BEGUM" is a registered trademark of Sansho Co., Ltd.), bentonite, polysorbate 80, aluminum monostearate, medicinal soap, sodium lauryl sulfate, lauromacrogol, and lecithin.

The types of suspensions that may be used herein are not limited and several kinds can be used, for example, acacia gum, sodium alginate, methylcellulose (MC), sodium carboxymethylcellulose, tragacanth, bentonite, polysorbate 80, polyvinylpyrrolidone, and aluminum monostearate.

The types of dispersants that may be used herein are not limited and several kinds can be used, for example, glycerin, sodium carboxymethylcellulose, sucrose solution, polysorbate 80, D-mannitol, and aluminum monostearate.

The types of aromatics that may be used herein are not limited and several kinds can be used, for example, lemon oil, orange oil, fruit juice extract such as lemon, orange, and pine, menthol, fennel oil, cinnamon oil, saffron, spearmint, mentha water, vanilla, peppermint oil, bergamot oil, rose oil, eucalyptus oil, and aromatic water.

The types of solubilizing agents that may be used herein are, for example, ethylenediamine, sodium benzoate, meglumine, and glycerin.

The types of coloring agents that may be used herein are not limited and several kinds can be used, for example, erythrosine (red No.3), rose bengale (red No.105), tartrazine (yellow No.4), fast green FCF (green No.3), indigocarmine (blue No. 2), all of which are tar color defined by the Health and Welfare Ministry, iron dioxide (yellow coloring agent), iron oxide (red coloring agent), caramel, β-carotene, bengala (Fe2O3), riboflavin, and medicinal carbon.

In order to bring out the function of rapid disintegration of a tablet to the fullest according to the present invention, it is desirable not to add a lubricant in a granulated material at all before compressing a granulated material obtained by mixing a mixture of a principal agent, a saccharide with high wettability against water, and a disintegrant with a binder including a saccharide with high wettability against water.

It is preferable to use an external lubricant spraying method in order to produce a tablet without adding a lubricant in a granulated material, without adhering a molding material on the punches and dies of a tabletting machine, and without causing tabletting problems such as sticking (refer to JP-A-56-14098, JP-A-59-205970, JP-A-3-9757, JP-A-4-295366, JP-A-5-318198, JP-A-8-281492, JP-A-8-19589, JP-A-7-124231).

Such a tablet is characterized in that the particles of the mixture are bound with a binder including a saccharide with high wettability against water without by a normal binder when a mixture of powder including a saccharide with high wettability against water and a disintegrant is granulated.

The tablet uses a saccharide with high wettability against water so that it can be rapidly disintegrated by the saliva in the mouth.

Furthermore, the particles of a granulated material are bound by a binder including a saccharide with high wettability against water. Accordingly, when the tablet is administered orally, a saccharide with high wettability against water in a binder is moistened by the saliva in the mouth and is dissolved or dispersed in the saliva. As a result, the binding force of the binder particles weakens and the granulated material is decayed so that the tablet is rapidly disintegrated.

Also, since the particles in the tablet are bound by a binder and since this binding force is strong, the tablet does not get chipped during storage and transportation.

Furthermore, according to specific embodiments of the present invention, a functional coating (for example an enteric coating) may be used with granules containing a principal agent in such a manner that the principal agent is dissolved in a targeted location, sustained release coating may be used so that the tablet is dissolved gradually, the solid dispersing granules may be prepared so as not to crystallize the principal agent, or the granules of principal agent may be dispersed in a wax matrix construction so that the pharmaceutical formulation may rapidly disintegrate in the mouth.

(2) The present invention relates to a tablet obtainable by binding a mixture powder including at least a principal agent, a saccharide with high wettability against water, a saccharide with high moldability, and a disintegrant by means of a binder including a saccharide with high wettability against water.

In such a tablet, the saccharide with high moldability is further added in a molding material according to the present invention considering the moldability of the tablet.

. At least one saccharide with high moldability selected from the group comprising lactose, maltitol, sorbitol, and oligosaccharide can be used.

With regards to the moldability and disintegration of a tablet in the mouth, the particle size of the saccharide with high moldability is preferably greater than or equal to 10 μm and less than or equal to 500 μm, more preferably greater than or equal to 20 μm and less than or equal to 300 μm, still more preferably greater than or equal to 20 μm and less than or equal to 200 μm.

Such a tablet uses a saccharide with high wettability against water so that it can rapidly disintegrate in the mouth.

Furthermore, the particle of granulated material is bound by a binder including a saccharide with high wettability against water. Accordingly, when the tablet is administered orally, the saccharide with high wettability against water in the binder is moistened by the saliva in the mouth and is dissolved or dispersed in the saliva. As a result, the binding force of the binder particles weakens and the granulated material is decayed so that the tablet is rapidly disintegrated.

Also, such a tablet advantageously includes particles of saccharide with high moldablity ensuring an excellent moldability for compressing. Therefore, the tablet does not get chipped when it is compressed and during storage and transportation.

Furthermore according to a specific embodiment of the present invention, a functional coating (for example an enteric coating) may be used with the granules containing a principal agent in such a manner that the principal agent is dissolved at a targeted location, a sustained release coating may be used to ensure a gradual dissolution of the tablet, a solid dispersion granule may be prepared to avoid crystallization of the principal agent, and the granules of principal agent may be dispersed in a wax matrix construction to avoid chipping the tablet during compression and during storage and transportation, thereby obtaining an intrabuccally rapidly disintegrable tablet.

(3) The present invention relates to a tablet characterized in that the ratio of the saccharide with high wettability against water and the saccharide with high moldability included in the granulated material of the present invention is such that the saccharide with high wettability against water is greater than or equal to 60 volume percentage and less than or equal to 90 volume percentage and the rest is saccharide with high moldability.

Namely, the volume ratio of the blended saccharide with high wettability against water and the saccharide with high moldability is within the range of 6:4 to 9:1.

More specifically, when the saccharide with high wettability against water and the saccharide with high moldability are extracted from several kinds of components included in the tablet and their sum volume is set as 100 volume%, the saccharide with high wettability against water is greater than or equal to 60 volume% and less than or equal to 90 volume% and the rest volume% is a saccharide with high moldability.

More preferably, the particles of saccharide with high wettability against water is greater than or equal to 60 volume% and less than or equal to 80 volume%, more preferably greater than or equal to 60 volume% and less than or equal to 70 volume%.

In such a tablet, the blend ratio of the particle of the wettable saccharide and the particle of the saccharide with high moldability is set so that a tablet having an excellent moldability of compression and being rapidly disintegrable in the mouth can be produced. Therefore, an intrabuccally rapidly disintegrable tablet can be produced at high productivity.

(4) The present invention relates to a tablet wherein the saccharide with high moldability according to specific embodiments of the present invention is at least one member selected from the group comprising lactose, maltitol, sorbitol, and oligosaccharide.

According to specific embodiments of the present invention, an available saccharide excellent in safety and moldability is selected as a saccharide with high moldability so as to easily produce a safe tablet highly moldable and intrabuccally rapidly disintegrable.

(5) The present invention relates to a tablet wherein the saccharide with high wettability against water used in tablets according to specific embodiments of the present invention is one member selected from the group comprising trehalose, mannitol, maltose, sorbitol, lactose, multitol, xylitol, sucrose, erythritol, and glucose.

According to such a tablet, a saccharide that is highly safe and wettable and is easily available is selected as a saccharide with high wettability against water, so as to easily produce a safe tablet that is intrabuccally rapidly disintegrable.

Furthermore, because the viscosity of the solution of saccharide with high wettability against water is not increased when it is dissolved in water, the water of the saliva is easily permeated in a tablet. The tablet is rapidly dissolved by saliva in the mouth because of this property.

(6) The present invention relates to a tablet wherein a surface active agent is included in a binder used in the tablet according to specific embodiments of the present invention.

Anionic surface-active agents, cationic surfactants, nonionic surfactants, amphoteric surfactants may be used as a surface active agents, as well as high molecular surface active agents such as Pluron or Poloxamer.

More concretely, preferable examples of anionic surface-active agents used according to the present invention are sulfates ($R \cdot O \cdot SO_3^- \cdot M^+$) such as sodium lauryl sulfate.

Preferable examples of nonionic surfactants are sorbitan esters and polysorbates, and polysorbate 80 more preferably example is.

A surface active agent having a HLB (hydrophile-lipophile balance) greater than or equal to 10 and less than or equal to 40 is preferable.

According to a specific embodiment of the present invention, the particles are bound by a binder including a surface active agent other than a saccharide with high wettability against water. The surface active agent lowers the tension of the water of the saliva thereby increasing the wettability of the binder in the mouth. The saccharide with high wettability against water is then rapidly moistened and dissolved or dispersed in the saliva from the binder. Accordingly the binding force of the binder is lost and the granulated material rapidly dissolved.

(7) The present invention relates to a tablet wherein a binder used for the tablet according to specific embodiments of the present invention is a water-soluble polymer.

The types of water-soluble polymer that may be used herein are not limited if they are soluble in water and harmless for the human body. Several kinds can be used, such as polyvinyl alcohol, polyethylene oxide, and polyvinylpyrrolidone.

According to specific embodiments of the present invention, the particles are bound by a water-soluble polymer including a saccharide with high wettability against water. Therefore, the binder is permeated by the water of the saliva when it comes into contact with it in the mouth. As a result, because the granulated material is quickly disintegrated and dispersed into particle sizes, the tablet can be rapidly disintegrated in the mouth.

Furthermore, according to specific embodiments of the present invention, the particles of saccharide with high wettability against water are dispersed in a water-soluble polymer binding the particles in the granulated material. Accordingly, the particles of saccharide with high wettability against water dispersed in the water-soluble polymer are dissolved into the saliva when they come into contact with it in the mouth. According to such a construction, when the tablet is in the mouth, the saccharide with high wettability against water in the binder is rapidly moistened by the saliva and becomes dissolved or dispersed in it. As a result, , the strength of the binder weakens so that the tablet becomes rapidly disintegrated comparing with a tablet where the particles within the granulated material are bound by a water-soluble polymer only.

(8) The present invention relates to a method for producing a tablet comprising the steps of: making a fluidized bed prepared by mixing a powdered mixture prepared by mixing at least a principal agent, a saccharide with high wettability against water, and a disintegrant homogenously with air; producing a granulated material including a principal agent by spraying an aqueous solution, in which a binder and a saccharide with high wettability against water are dissolved, into the fluidized powdered mixture and drying the granulated material; and compressing the granulated material including the principal agent to be tabletted.

According to such a production method, an intrabuccally rapidly disintegrable tablet can be produced by means of a fluid bed granulation method and a compression mold method which are generally used for producing a normal tablet so that no new or special apparatus is required for producing an intrabuccally rapidly disintegrable tablet.

In tablets produced by such a production method, a granulated material is bound by a binder including a saccharide with high wettability against water. Therefore, its disintegrability is superior in the mouth comparing with that of a tablet in which granulated material is compressed using only a binder.

(9) The present invention relates to a method of producing a tablet comprising the steps of; making a fluidized bed by mixing a powdered mixture prepared by mixing at least a principal agent, a saccharide with high wettability against water, a saccharide with high moldability, and a disintegrant homogenously with air; producing a granulated material including a principal agent by spraying an aqueous solution in which a binder and a saccharide with high wettability against water are dissolved into the fluidized powdered mixture and drying the granulated material; and compressing the granulated material including the principal agent to be tabletted.

Additionally, according to this production method, an intrabuccally rapidly disintegrable tablet can be produced by means of a conventional fluid bed granulation method and a conventional compression molding method so that no new and special apparatus is required for producing an intrabuccally rapidly disintegrable tablet.

In the tablet produced by such a production method, the particles in the granulated material are bound by a binder including a saccharide with high wettability against water. Its disintegrability in the mouth is therefore superior to that of a tablet wherein the granulated material is compressed using a binder including only a water-soluble polymer.

Furthermore, according to this production method, the particles of saccharide with high moldability are included in a granulated material. Tabletting problems such as sticking are thereby avoided.

(10) The present invention also relates to a method for producing a tablet, wherein a surface active agent is further added in the aqueous solution including a binder and a saccharide with high wettability against water according to a specific embodiment of the present invention.

According to this specific embodiment, a surface active agent is added in the binder. In tablets produced according to this method, the particles of the granulated material are bound by a binder including a surface active agent other than a saccharide with high wettability against water so that this tablet disintegrates more rapidly in the mouth.

(11) The present invention also relates to a method for producing a tablet wherein the binder used according to specific embodiments of the present invention is a water-soluble polymer.

According to this specific production method, a water-soluble polymer is used as a binder and the binder is dissolved in the water of the saliva when the tablet produced by this production method comes into contact with the saliva in the mouth. The granulated material is thereby quickly dissolved and dispersed into particle sizes and rapidly disintegrated in the mouth.

Furthermore in tablets produced according to such method, the particles of saccharide with high wettability against water are dispersed in a water-soluble polymer binding the particles in the granulated material. Therefore, when this tablet comes into contact with the saliva in the mouth, the particles of saccharide with high wettability against water dispersed in a water-soluble polymer are dissolved in the saliva. In such a structure, when the tablet is taken orally, the saccharide with high wettability against water in the binder quickly moistens and dissolves or disperses in the saliva. Therefore, the binding force of the particles in the binder weakens and the granulated material disintegrates and therefore the tablet rapidly disintegrates.

(12) The present invention also relates to a method for producing a tablet wherein the aqueous solution including a binder and a saccharide with high wettability against water according to a specific embodiment of the present invention is adjusted in such a manner that the binder is greater than or equal to 1 volume and less than or equal to 3 volumes for 100 volumes of water and the volume of the saccharide with high wettability against water is greater than or equal to 5 volumes and less than or equal to 6 volumes for 100 volumes of water.

According to this specific embodiment, the ratio of the binder and the saccharide with high wettability against water included in the aqueous solution used for the granulation is adjusted so that the compressed tablet has an appropriate hardness and the tablet is rapidly dissolved in the mouth. An intrabuccally rapidly disintegrable tablet which does not or rarely get chipped during storage and transportation and rapidly dissolves in the mouth can be produced.

Brief Description of Drawings

Fig.1 is an explanatory view diagrammatically showing one preferred embodiment of a granulated material used in the tablet (an intrabuccally rapidly disintegrable tablet) of the present invention.

Fig.2 is a process drawing schematically showing one embodiment of a method of manufacturing the tablet (an intrabuccally rapidly disintegrable tablet) of the present invention.

Fig.3 is a process drawing schematically showing one embodiment of a method of manufacturing the tablet (an intrabuccally rapidly disintegrable tablet) of the present invention.

Fig.4 is an explanatory view diagrammatically showing how the tablet (an intrabuccally rapidly disintegrable tablet) Ta of the present invention is disintegrated in the mouth. Fig.4(a) is a perspective view of the tablet (an intrabuccally rapidly disintegrable tablet), Fig.4(b) is a diagrammatic view in which the area R1 in Fig.4(a) is enlarged, Fig.4(c) is a diagrammatic view in which the area R2 in Fig.4(b) is enlarged, and Fig.4(d) is a diagrammatic view showing how the tablet (intrabuccally rapidly disintegrable tablet) is disintegrated in the saliva.

Fig.5 is an explanatory view diagrammatically showing another preferred embodiment of a granulated material used in the tablet (intrabuccally rapidly disintegrable tablet) of the present invention.

Fig.6 is a process drawing schematically showing one embodiment of a method of manufacturing of the tablet (an intrabuccally rapidly disintegrable tablet) of the present invention.

Fig.7 is a process drawing schematically showing one embodiment of a method of manufacturing the tablet (an intrabuccally rapidly disintegrable tablet) of the present invention

Fig.8 diagrammatically shows the disintegration steps of the tablet (an intrabuccally rapidly disintegrable tablet) in the mouth. Fig.8(a) is a perspective view of the tablet (an intrabuccally rapidly disintegrable tablet), Fig.8(b) is a diagrammatic view in which the area R3 in Fig.8(a) is enlarged, Fig.8(c) is a diagrammatic view in which the area R4 in Fig. 8(b) is enlarged, and Fig.8(d) is a diagrammatic view showing how the tablet (intrabuccally rapidly disintegrable tablet) is disintegrated in the saliva.

Fig.9 is an explanatory view diagrammatically showing still another preferred embodiment of a granulated material used in the intrabuccally rapidly disintegrable tablet of the present invention.

Fig.10 is a process drawing schematically showing another embodiment of a method of manufacturing the tablet (intrabuccally rapidly disintegrable tablet) of the present invention.

Fig.11 is a process drawing schematically showing another embodiment of a method of manufacturing the tablet (intrabuccally rapidly disintegrable tablet) of the present invention.

Fig.12 diagrammatically shows a disintegration procedure of the tablet (intrabuccally rapidly disintegrable tablet) in the mouth. Fig.12(a) is a perspective view of the tablet (an intrabuccally rapidly disintegrable tablet), Fig.12(b) is a diagrammatic view in which the area R5 in Fig.12(a) is enlarged, Fig.12(c) is a diagrammatic view in which the area R6 in Fig.12(b) is enlarged, and Fig.12(d) is a diagrammatic view showing how the tablet (an intrabuccally rapidly disintegrable tablet) is disintegrated in the saliva.

Fig.13 shows a diagrammatic entire construction of an external lubricant spraying type tabletting machine used for manufacturing the tablet of the present invention.

Fig.14 is a plane view of a rotary-type tabletting machine of the external lubricant spraying type tabletting machine in Fig.13

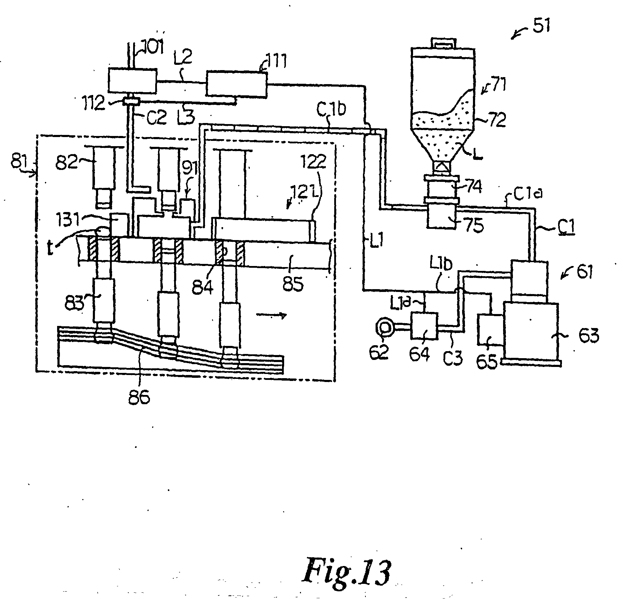

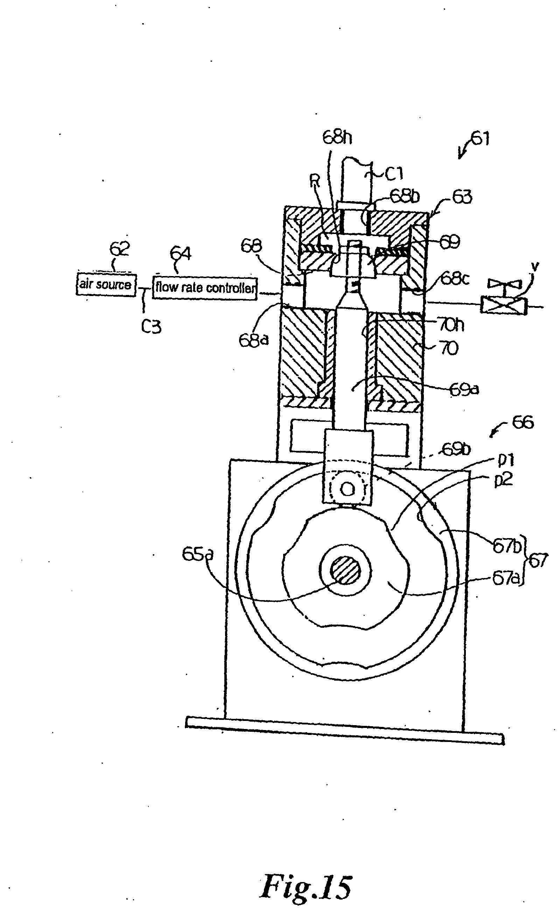

. Fig.15 is an explanatory view of a pulsating vibration air generation means comprising the external lubricant spraying type tabletting machine in Fig.13

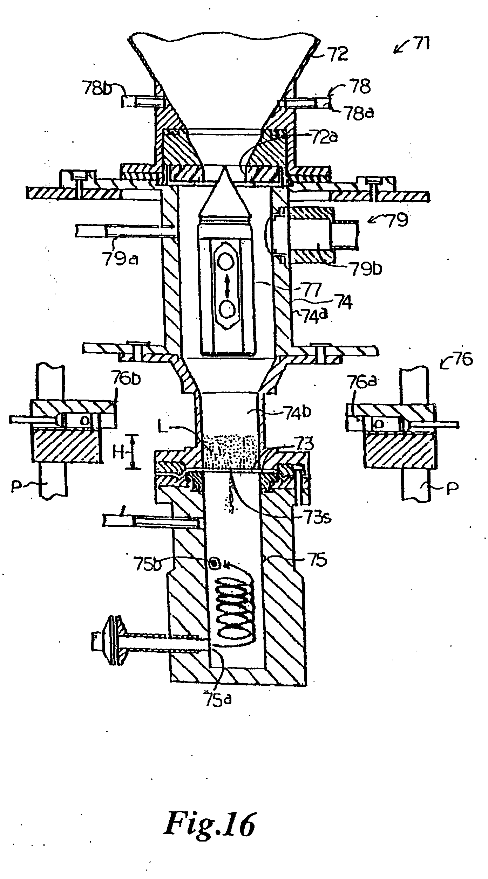

. Fig.16 is a diagrammatic sectional view of discharge means (quantitative feeder) comprising the external lubricant spraying type tabletting machine in Fig.13.

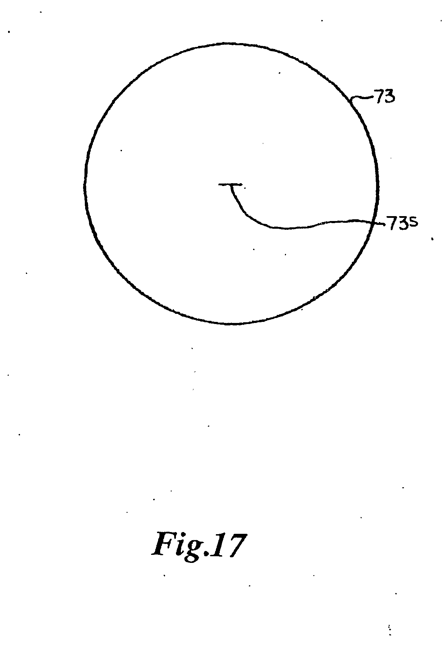

Fig.17 is a plane view diagrammatically showing an elastic membrane used for the discharge means (quantitative feeder) in Fig.16.

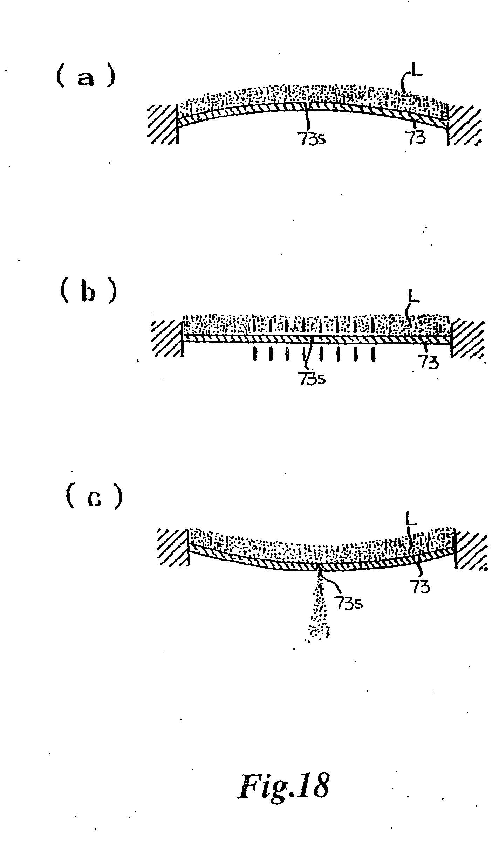

Fig.18 is a view explaining operations of the elastic membrane in Fig.17.

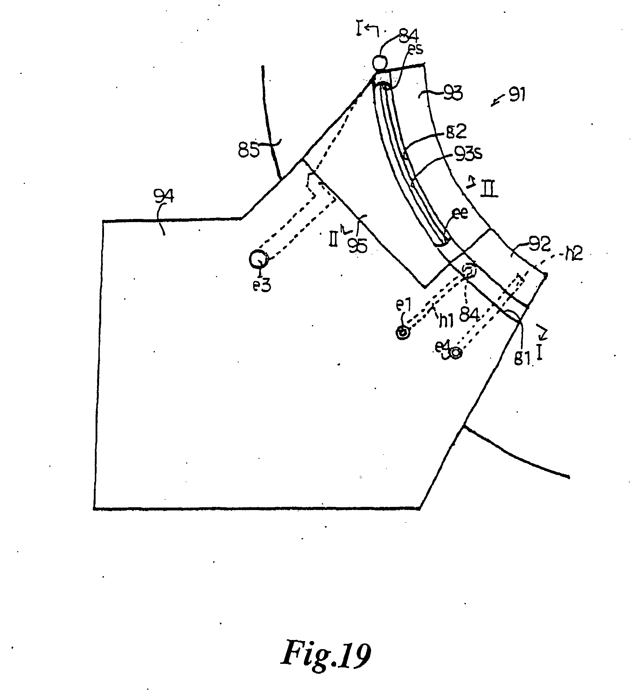

Fig.19 is a plane view diagrammatically showing the construction of a lubricant spraying means comprising the external lubricant spraying type tabletting machine in Fig.13.

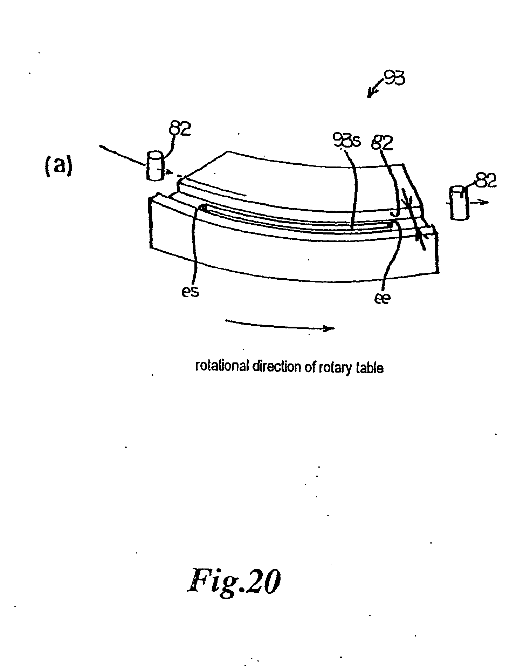

Fig.20 is an outer perspective view diagrammatically showing an upper punch lubrication means of the lubricant spraying means in Fig.19 when seen from the periphery of the rotary table into the center thereof.

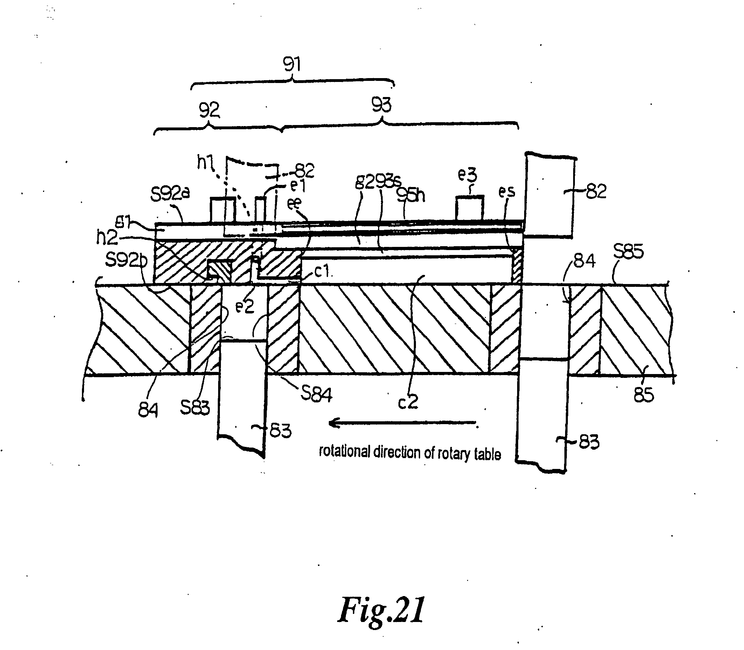

Fig.21 shows a diagrammatic section along the line I-I in Fig.19.

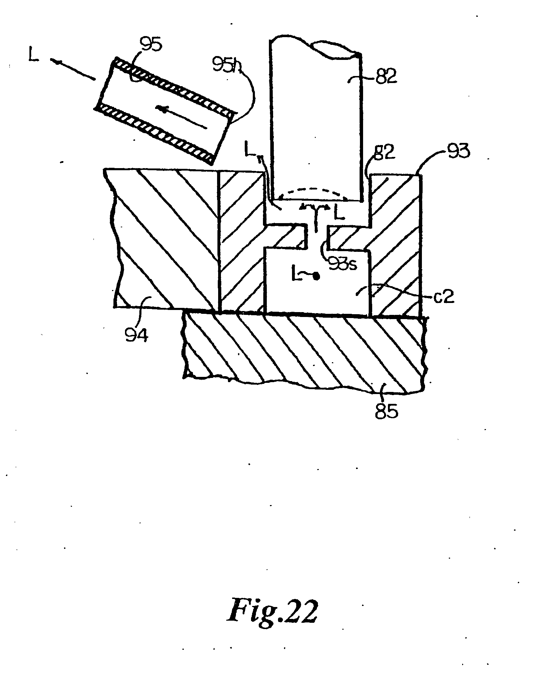

Fig.22 shows a diagrammatic section along the line II-II in Fig.19.

Best Mode for Carrying Out the Invention

(Embodiment of the Invention. 1)

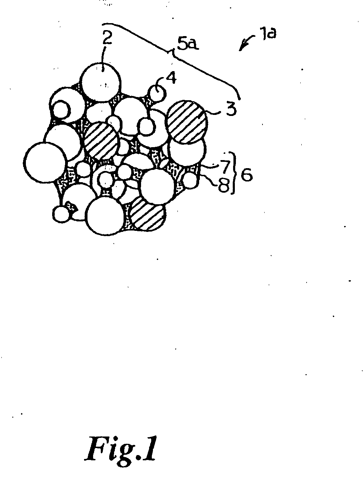

Fig.1 is an explanatory view diagrammatically showing one preferred embodiment of a granulated material used in the tablet (intrabuccally rapidly disintegrable tablet) of the present invention.

A granulated material 1a is constituted of a mixed powder particles 5a including a principal agent particle 2 ···, a saccharide particle 3 ··· with high wettability against water, and a disintegrant particle 4 ··· , bound by a binder 6 ··· including a water-soluble polymer 7 and a particle (precipitation of fine particle) 8 ··· of a saccharide with high wettability against water.

More specifically, the binder 6 is so constructed that the particle (precipitation of fine particles) 8 ··· of saccharide with high wettability against water is dispersed in a water-soluble polymer 7.

When a binder solution supplemented with particles (precipitation of fine particle) 8 ··· of a saccharide with high wettability against water is dried, the saccharide with high wettability against water included in the solution is separated out to constitute particles (precipitation of fine particles) 8 ··· of a saccharide with high wettability against water.

The principal agent (particle) 2 according to the present invention may be constituted of a particle or a granule including an active agent, a granule having medicinal properties coated with a functional coating, a granule comprising an active agent dispersed in a wax matrix construction, or a solid dispersion granule.

The particle diameter of the principal agent 2 is greater than or equal to 10 µm and less than or equal to 500 µm, more preferably greater than or equal to 20 µm and less than or equal to 300 µm, still more preferably greater than or equal to 20 µm and less than or equal to 200 µm.

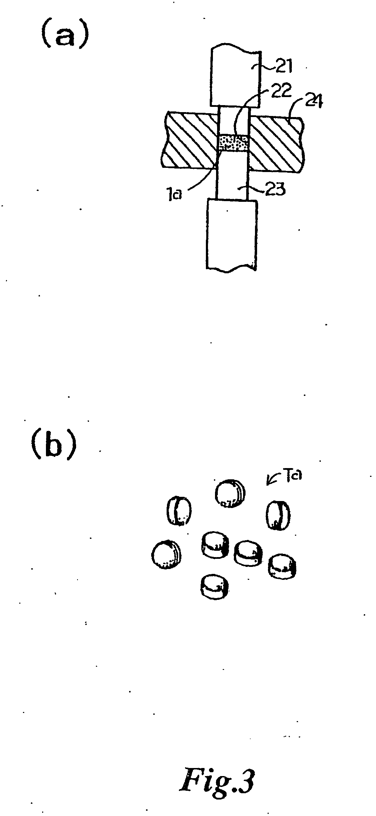

A granulated material 1a using a principal agent particle 2 within the above-mentioned range is easily tabletted and the tablet (Ta shown in Fig.3) produced by compressing such granulated material 1a disintegrates well in the mouth.

The saccharide particle 3 with high wettability against water according to the present invention may be at least one member selected from the group comprising trehalose, mannitol, maltose, sorbitol, lactose, multitol, xylitol, sucrose, erythritol, and glucose.

The particle diameter of a saccharide particle 3 with high wettability against water is also greater than or equal to 10 µm and less than or equal to 500 µm, more preferably greater than or equal to 20 µm and less than or equal to 300 µm, still more preferably greater than or equal to 20 µm and less than or equal to 200 µm.

The granulated material 1a comprising a saccharide particle 3 with high wettability against water within the above-mentioned range is easily tabletted and the tablet Ta produced by compressing such granulated material 1a disintegrates well in the mouth.

The disintegrant particle 4 according to the present invention may be selected from the group consisting of Sodium alginate, carmellose, sodium carboxymethyl-cellulose, calcium carboxymethyl-cellulose, agar powder, gelatin, shellac, crystalline cellulose, calcium carbonate, sodium bicarbonate, starch such as corn starch and potato starch, sodium starch glycolate, tragacanth, methylcellulose (MC), pregelatinized starch (HPS), bentonite, sodium lauryl sulfate, calcium phosphate and povidone.

The particle diameter of the disintegrant particle 4 is also greater than or equal to 10 μm and less than or equal to 500 μm, more preferably greater than or equal to 20 μm and less than or equal to 300 μm, still more preferably greater than or equal to 20 μm and less than or equal to 200 μm.

The granulated material 1a using a disintegrant particle 4 within the above-mentioned range is easily tabletted and the tablet Ta produced by compressing such granulated material 1a disintegrates well in the mouth.

The granulated material 1a is characterized in that the mixed powder particles 5a including a principal agent particle 2, a saccharide particle 3 with high wettability against water, and a disintegrant particle 4, are bound by a binder 6 including a particle (precipitation of fine particles) 8 ··· of a saccharide with high wettability against water.

. The types of water-soluble polymers 7 that may be used for the binder 6 according to the present invention are not limited as long as they are normal water-soluble polymers. Polyvinyl alcohol (PVA), hydroxypropylcellulose (HPC), and hydroxypropyl-methylcellulose (HPMC) or a combination thereof may for instance be used in accordance with the present invention.

The types of saccharides with high wettability against water that may be included as particles 8(precipitation of fine particle) in the binder 6 in accordance with the present invention include at least one member selected from the group comprising trehalose, mannitol, maltose, sorbitol, lactose, multitol, xylitol, sucrose, erythritol, and glucose.

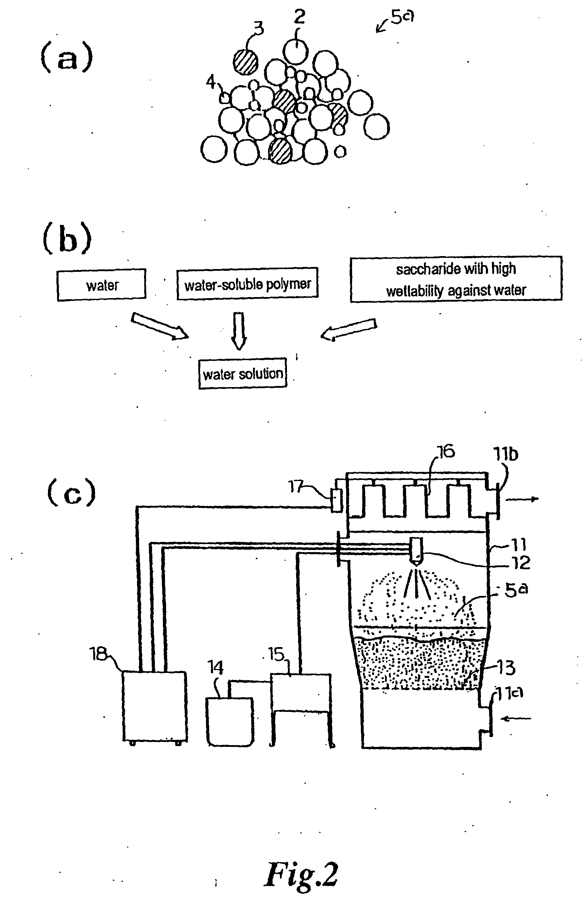

Fig.2 and Fig.3 are process drawings schematically showing one embodiment of a method of manufacturing the intrabuccally rapidly disintegrable tablet of the present invention.

In the method in Fig.2(a), a mixed powder particle 5a is prepared by homogeneously mixing principal agent particles 2, particles 3 of saccharide with high wettability against water and disintegrant particles 4 with a mixer

. Then, a binder solution for granulating the mixed powder particles 5a is prepared as shown in Fig. 2(b).

The binder solution for granulating the mixed powder particles 5a is prepared so that the water-soluble polymer and the saccharide with high wettability against water are dissolved in water.

More particularly, the binder solution used for granulating the mixed powder particles 5a is adjusted in such a manner that the volume of water-soluble polymer is greater than or equal to 1 volume% and less than 3 volume% for 100 volume% of water and the volume of saccharide with high wettability against water is greater than or equal to 5 volume% and less than or equal to 6 volume% for 100 volume% of water.

When the aqueous solution includes a volume greater than or equal to 3 volume% of water-soluble polymer(the density of the binder solution used for a normal tablet becomes such that the volume of water-soluble polymer is greater than or equal to 3 volume% and less than or equal to 5 volume%), the binding force between the principal agent particle 2 ···, the saccharide particle 3 with high wettability against water and the disintegrant particle 4 ···, in the granulated material 1a, becomes similar to that in a conventional tablet and the disintegration speed in the mouth is slower than that existing in tablets wherein the volume of water-soluble polymer is adjusted to be less than 3 volume%.

If the water-soluble polymer 1 in the binder solution used for granulating the mixed powder 5a constitutes less than 1 volume%, the binding force between the principal agent particle 2 ···, the saccharide particle 3 with high wettability against water and the disintegrant particle 4 ···, comprised in the granulated material, weakens as it does when no water-soluble polymer at all is used in the aqueous solution for granulating the mixed powder 5a (this is the case if for example when only water or ethanol is used as binder). Therefore, the moldability of compression decreases and the hardness of the compressed tablet becomes lower than is desirable so that the tablet may easily chip during storage and transportation.

As shown in Fig.2(c), the mixed powder of particles 5a obtained in the procedure in Fig.2(a) is stored in a granulation tank 11 of a fluid layer granulation means and is fluidized by being mixed with heated air according to a conventional method. Then, the aqueous solution in which a water-soluble polymer and a saccharide with high wettability against water are added and dissolved is sprayed from a spraying means 12 onto the fluidized mixed powder of particles 5a. They are dried and produced to be a granulated material having a specific particle size distributions (aggregation of granulated material 1a shown in Fig.1).

In Fig.2(c), the numeral 11a shows a heated air supply port, and 11b is a discharge port for discharging outside heated air supplied in the granulation tank 11.

Furthermore, the numeral 13 shows a porous screen, 14 is a binder solution tank, 15 is a binder solution supply means for supplying a binder solution stored in the binder solution tank 14 to the spraying means 12, 16 is a dust collection filter, 17 is a dust collection filter vibration means for vibrating the dust collection filter 16 for dropping the powder, the granulated material, or the material under granulation attached on the dust collection filter 16, 18 is an air source such as a blower for supplying compressed air for spraying a binder solution from the spray means 12 and supplying a compressed air for driving the dust collection filter vibration means.

Then, in the procedure shown in Fig.3(a), a thus produced granulated material 1a is compressed and molded by means of an upper punch 21, a die 22, and a lower punch 23 of a rotary tabletting machine so as to produce an intrabuccally rapidly disintegrable tablet Ta (See Fig.3(b)).

Prior to performing the procedure shown in Fig. 3 (a), lubricant may be added in the granulated material in order to achieve smooth and continuous tabletting .

Alternatively, lubricant may be coated on each surface of the upper punch 21, the die 22, and the lower punch 23 of the tabletting machine so that granulated material 1a ··· may be compressed by means of the thus lubricated upper punch 21, die 22, and lower punch 23 without adding a lubricant in the granulated material 1a.

. Furthermore, one or more members selected from the group consisting of acidifiers such as citric acid, tartaric acid, and malic acid, foaming agents such as baking soda, artificial sweeteners such as dipotassium glycyrrhizinate, aspartame, stevia, and thaumatin, perfumes such as lemon, lemon lime, orange, and menthol, coloring agents such as food yellow No.5, food red No. 2, food blue No.2 may be added if required in the production of a mixed powder of particles 5a.

The member shown as 24 in Fig.3(a) is a part of a rotary table of the rotary type tabletting machine.

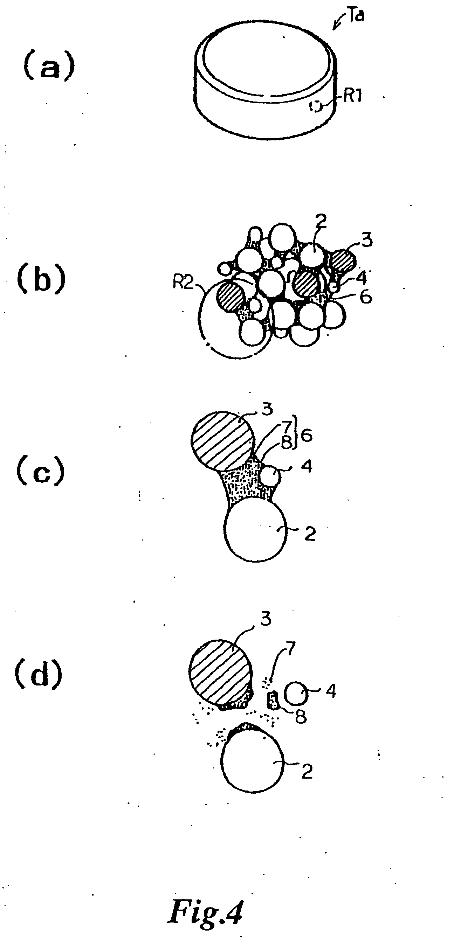

Fig.4 is an explanatory view diagrammatically showing how the tablet Ta (intrabuccally rapidly disintegrable tablet) is disintegrated in the mouth. Fig.4(a) is a perspective view of the tablet Ta (intrabuccally rapidly disintegrable tablet), Fig.4(b) is a diagrammatic view in which the area R1 in Fig.4(a) is enlarged, Fig.4(c) is a diagrammatic view in which the area R2 in Fig.4(b) is enlarged, and Fig.4(d) is a diagrammatic view showing how the tablet Ta (intrabuccally rapidly disintegrable tablet) is disintegrated in saliva.

In this tablet Ta (intrabuccally rapidly disintegrable tablet), the binding between the particles 2 ···, 3 ···, 4 ··· within granulated material 1a ··· included in the tablet Ta is achieved by a binder 6 including a water-soluble polymer 7 and particles (precipitation of fine particle) 8 ··· of saccharide with high wettability against water.

. Therefore, when the tablet Ta does not come into contact with water, the particles 2 ···, 3 ···, 4 ··· are bound by a water soluble polymer 7 so that each granulated material 1a does not brake easily as is the case in a conventional tablet. For this reason, the tablet Ta does not get chipped easily during storage and transportation.

At the same time and as shown in Fig.4(d), when the tablet Ta is taken orally, the disintegrant 4 becomes swollen by the water in the saliva and the tablet is disintegrated into the particle size constituting the granulated material.

The fact that the tablet is disintegrated into the particles constituting the granulated material 1a by a disintegrant 4 is the same as the prior intrabuccally rapidly disintegrable tablet. However according to the tablet Ta, the particle (precipitation of fine particle) 8 ··· of saccharide with high wettability against water is dispersed therein. Accordingly, the tablet Ta is easily moistened in the water of the saliva in the mouth as compared with a tablet comprising a binder 6 consisting only of a water-soluble polymer 7 (i.e. the particle (precipitation of fine particle) 8 ··· of saccharide with high wettability against water is not included in the binder 6). Thereby, the particle (precipitation of fine particle) 8 ··· of saccharide with high wettability against water becomes moistened by the water of the saliva in the mouth, is then dispersed or dissolved therein, and then the physical strength of the water-soluble polymer 7 weakens and the surface area of the water-soluble polymer 7 is enlarged. As a result, the water-soluble polymer 7 rapidly becomes permeated by the water of the saliva in the mouth and is dispersed and dissolved therein.

Furthermore, according to a specific embodiment of the present invention, the saccharide particle 3 with high wettability against water is used as a particle of the granulated material 1a and as a particle (precipitation of fine particle) 8 ··· of saccharide with high wettability against water dispersed in a binder 6. The particles of saccharide with high wettability against water included in the granulated material 1a ··· is also dissolved or dispersed in saliva so that the tablet Ta (intrabuccally rapidly disintegrable tablet) is quickly disintegrated in the mouth.

Furthermore, when the saccharide particles 3 with high wettability against water and the saccharide particles (precipitation of fine particle) 8 with high wettability against water are dissolved in saliva, its viscosity does not become higher than that of water $\eta$ w. Thereby, when the saccharide particles 3 with high wettability against water and the saccharide particles (precipitation of fine particle) 8 with high wettability against water are dissolved in saliva, its viscosity remains such that it may quickly permeate the tablet Ta. The tablet Ta may thereby rapidly disintegrate in the mouth.

(Embodiment of the Invention 2)

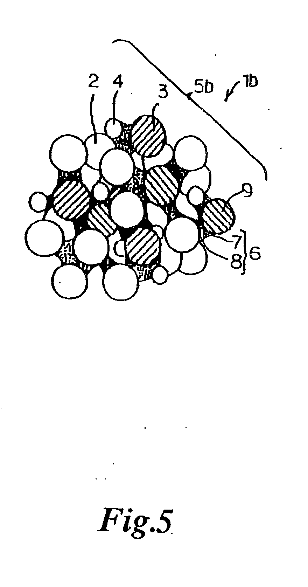

Fig.5 is an explanatory view diagrammatically showing another preferred embodiment of a granulated material used in the tablet (intrabuccally rapidly disintegrable tablet) of the present invention.

The structure of the granulated material 1b is similar to that of the granulated material 1a described in the embodiment of the invention 1 except that the granulated material 1b further comprises a saccharide particle with high moldability 9 ···.

The granulated material 1b comprises mixed particles 5b including a principal agent particle 2 ···, a saccharide particle 3 with high wettability against water, a disintegrant particle 4 ···, and a saccharide particle with high moldability 9 ··· bound by a binder 6 ··· including a water-soluble polymer 7 and a saccharide particle (precipitation of fine particle) 8 with high wettability against water.

The binder 6 is prepared so that the saccharide particles (precipitation of fine particle) 8 ··· with high wettability against water are dispersed in the water soluble polymer 7.

The principal agent particle 2 may be constituted of a granule including an active agent or a granule having medicinal properties coated with a functional coating, a granule in which an active agent is dispersed in a wax matrix construction, or a solid dispersion granule.

The saccharide particles 9 with high moldability of the present invention may be a member selected from the group comprising lactose, maltitol, sorbitol, and oligosaccharide.

The particle diameter of the saccharide particle with high moldability 9 is greater than or equal to 10 μm and less than or equal to 500 μm, more preferably greater than or equal to 20 μm and less than or equal to 300 μm, still more preferably greater than or equal to 20 μm and less than or equal to 200 μm.

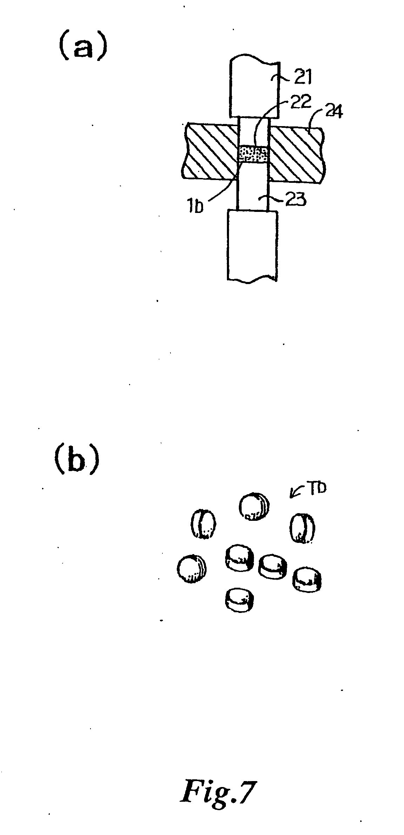

Granulated material 1b comprising a saccharide with high moldability particle 9 within the above-mentioned range is easily tabletted and the tablet (Tb shown in Fig.7) produced by compressing such granulated material 1b possesses a high effectiveness for disintegrating in the mouth.

The types of saccharide 3 that can be used in this embodiment are the same as those described for the granulated material 1a.

The ratio of the saccharide particles 3 with high wettability against water and the saccharide particles with high moldability 9 is such that the volume of saccharide particles 3 with high wettability against water is preferably greater than or equal to 60 volume percentage and less than or equal to 90 volume percentage, more preferably greater than or equal to 60 volume percentage and less than or equal to 80 volume percentage, still more preferably greater than or equal to 60 volume percentage and less than or equal to 70 volume percentage when the sum of the saccharide particles 3 with high wettability against water and the saccharide particles with high moldability 9 is set at 100 volume%.

If the volume of saccharide particles with high moldability 9 exceeds 40 volume%, tabletting problems during compression (for example sticking, laminating or capping) may be reduced, but the disintegration speed in the mouth decreases compared to that of a tablet wherein such volume is less than or equal to 40 volume%.

If the volume of saccharide particles with high moldability 9 is less than 10 volume%, the disintegration in the mouth is the same as that when the volume is greater than or equal to 10 volume%. However, the moldability during compression decreases compared to that when the volume is greater than or equal to 10 volume%.

The disintegrants 4 that can be used in this specific embodiment are the same as those described for the granulated material 1a.

The granulated material 1b is characterized in that the mixed powder of particles 5b including a principal agent particle 2, a saccharide particle 3 with high wettability against water, a saccharide particle with high moldability 9 and a disintegrant particle 4 bound by a binder 6 including a water-soluble polymer 7 and a saccharide particle (precipitation of fine particle) 8 with high wettability against water.

The types of water-soluble polymers 7 that can be used in this specific embodiment are the same as those described for the granulated material 1a.

The types of saccharide particle (precipitation of fine particle) 8 with high wettability against water used for the binder 6 that can be used in this specific embodiment are the same as those described for the granulated material 1a.

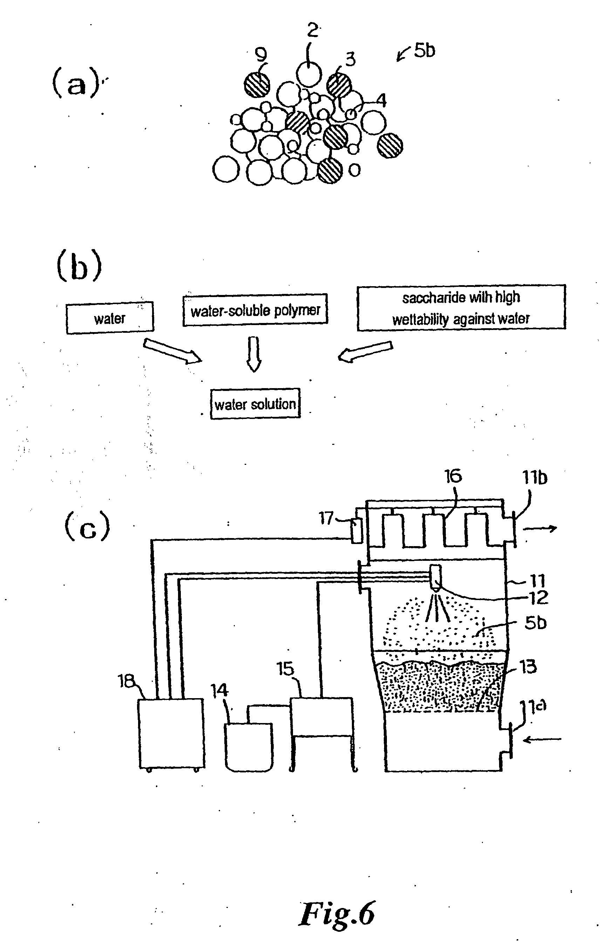

Fig.6 and Fig. 7 are process drawings schematically showing one embodiment of a manufacturing method of the intrabuccally rapidly disintegrable tablet of the present invention.

. In the method in Fig.6(a), a mixed powder of particles 5b is prepared by homogeneously mixing a principal agent particle 2, a saccharide particle 3 with high wettability against water, a disintegrant particle 4; and a saccharide particle with high moldability 9 by a mixer.

The ratio of saccharide particles 3 with high wettability against water and saccharide particles with high moldability 9 is adjusted in such a manner that the volume of saccharide particles 3 with high wettability against water is greater than or equal to 60 volume% and less than or equal to 90 volume%.

The binder solution for granulating the mixed powder of particles 5b is prepared as shown in Fig.6(b).

The binder solution for granulating the mixed powder of particles 5b is the same as that described for the embodiment of the invention 1.

As shown in Fig. 6 (c), the mixed powder of particles 5b obtained in the method in Fig.6(a) is stored in a granulation tank 11 of a fluid layer granulation means and is fluidized by being mixed with heated air according to a conventional method. An aqueous solution wherein a water-soluble polymer and a saccharide with high wettability against water are dissolved is then prepared as shown in Fig.6(b) and is sprayed from a spraying means 12 to the fluidized mixed powder of particles 5b, they are then dried and transformed into a granulated material having the desired particle size and distribution (a granulated material 1b shown in Fig.5).

Then, in the procedure shown in Fig.7(a), the thus produced granulated material 1b is compressed and molded and tablets Tb (intrabuccally rapidly disintegrable tablet) are produced (See Fig.7(b)).

Prior to performing the procedure shown in Fig. 7(a), a lubricant may be added in the granulated material 1b in order to provide for a smooth and continuous tabletting.

Alternatively, a lubricant may be applied on each surface of the upper punch 21, the die 22, and the lower punch 23 of the tabletting machine whereby the granulated material 1b maybe compressed by the thus lubricated upper punch 21, die 22, and lower punch 23 without adding a lubricant in the granulated material 1b.

Furthermore, one or more members selected from the group consisting of acidifiers such as citric acid, tartaric acid, and malic acid, foaming agents such as baking soda, artificial sweeteners such as dipotassium glycyrrhizinate, aspartame, stevia, and thaumatin, perfumes such as lemon, lemon lime, orange, and menthol, coloring agents such as food yellow No.5, food red No. 2, food blue No.2 may be added if required in the production of the mixed powder of particles 5b.

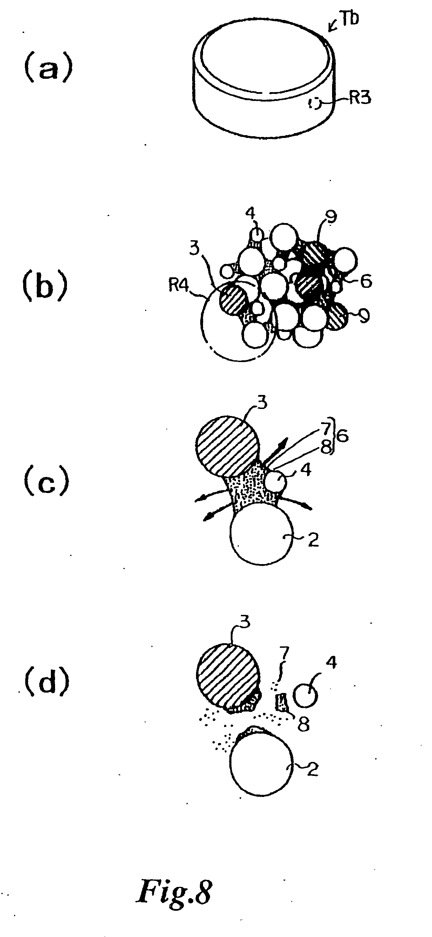

Fig.8 is an explanatory view diagrammatically showing how the tablet Tb (intrabuccally rapidly disintegrable tablet) is disintegrated in the mouth. Fig.8(a) is a perspective view of the intrabuccally rapidly disintegrable tablet Tb, Fig.8(b) is a diagrammatic view in which the area R3 in Fig.8(a) is enlarged, Fig.8(c) is a diagrammatic view in which the area R4 in Fig.8(b) is enlarged, and Fig.8(d) is a diagrammatic view showing how the tablet Tb (intrabuccally rapidly disintegrable tablet) is disintegrated in saliva.

In this tablet Tb (intrabuccally rapidly disintegrable tablet), the binding between the particles 2 ···, 3 ···, 4 ···, 9 ··· in the granulated material 1b included in the tablet Tb is achieved by the water-soluble polymer 7.

Therefore, when the tablet Tb does not come into contact with water, the particles 2 ···, 3 ···, 4 ···, 9 ··· are bound by a water soluble polymer 7 so that each granulated material 1b is hard and its resistance to chipping is comparable to that of a conventional tablet. The tablet Tb is not chipped easily during storage and transportation.

Furthermore, the addition of a saccharide particle with high moldability 9 in the granulated material 1b, reduces problems such as sticking, laminating, and capping during the compression and tabletting of the granulated material 1b

. Additionally and as shown in Fig.8(d), when the tablet Tb is inserted in the mouth, the saccharide particles (precipitation of fine particle) 8 with high wettability against water are dispersed in a binder 6 similarly as in the tablet Ta. Accordingly, the tablet Tb (intrabuccally rapidly disintegrable tablet) is rapidly disintegrated in the mouth through the same action as in the tablet Ta.

Furthermore, when the saccharide particles 3 with high wettability against water and the saccharide particles (precipitation of fine particle) 8 with high wettability against water are dissolved in the saliva, its viscosity does not become higher than that of water $\eta$ w. Thereby, when the saccharide particles 3 with high wettability against water and the saccharide particles (precipitation of fine particle) 8 with high wettability against water are dissolved in saliva, its viscosity remains such that it may quickly permeate the tablet Tb. The tablet Tb thereby rapidly disintegrates in the mouth.

(Embodiment of the Invention 3)

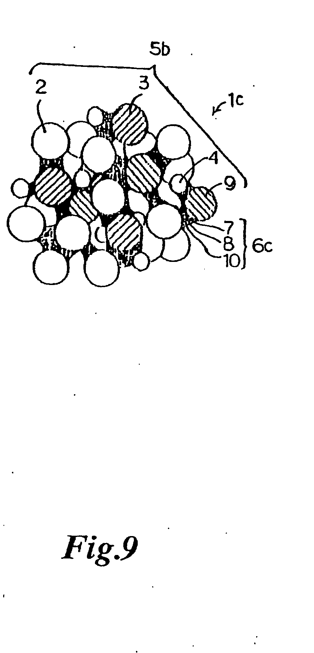

Fig. 9 is an explanatory view diagrammatically showing another preferred embodiment of a granulated material used in the intrabuccally rapidly disintegrable tablet of the present invention.

. The granulated material 1c has a similar structure as that of the granulated material 1b shown in Fig.5 but differs as follows

. The granulated material 1c comprises a binder 6c for binding a mixed powder of particles 5b including a principal agent particle 2, a saccharide particles 3 with high wettability against water, a disintegrant particle 4, and a saccharide particle with high moldability 9.

The granulated material 1c is such that the particles 2 ···, 3 ···, 4 ···, 9 ··· in the mixed powder of particles 5b are bound by a binder 6c wherein saccharide particles (precipitation of fine particle) 8 with high wettability against water and a surface active agent 10 ··· are dispersed in a water-soluble polymer 7.

Anionic surface-active agents, cationic surfactants, nonionic surfactants, amphoteric surfactant may be used as a surface active agents 10, as well as high molecular surface active agents such as Pluron or Poloxamer.

More concretely, preferred examples of anionic surface-active agents are sulfates ($R \cdot O \cdot SO_3^- \cdot M^+$) such as sodium lauryl sulfate.

preferred examples of nonionic surfactants are sorbitan esters and polysorbates, and more preferred is polysorbate 80.

A surface active agent in which the HLB (hydrophile-lipophile balance) is greater than or equal to 10 and less than or equal to 40 is preferred. Such surface active agents such as polyoxyethylene (20) sorbitan monooleate (HLB=15.0), Polyoxy-ethylene (20) sorbitan monopalmitate) (HLB=15.6), Polyoxyethylene (20) sorbitan monolaurate (HLB=16.7), Polyoxyethylene (30) sorbitan stearate (HLB=16.0), Polyoxyethylene (40) stearate (HLB=16.9), Polyoxyethylene (100) stearate (HLB=18.8), triethanol-amine oleate (HLB=12.0), Sodium oleate (HLB=18.0), and sodium lauryl sulfate (HLB=40) have this HLB.

The suitable ratio of surface active agent 10 to water-soluble polymer 7 is determined by experiments analysing the strength of the binder 6c and the wettability of the binder 6c for saliva (namely water).

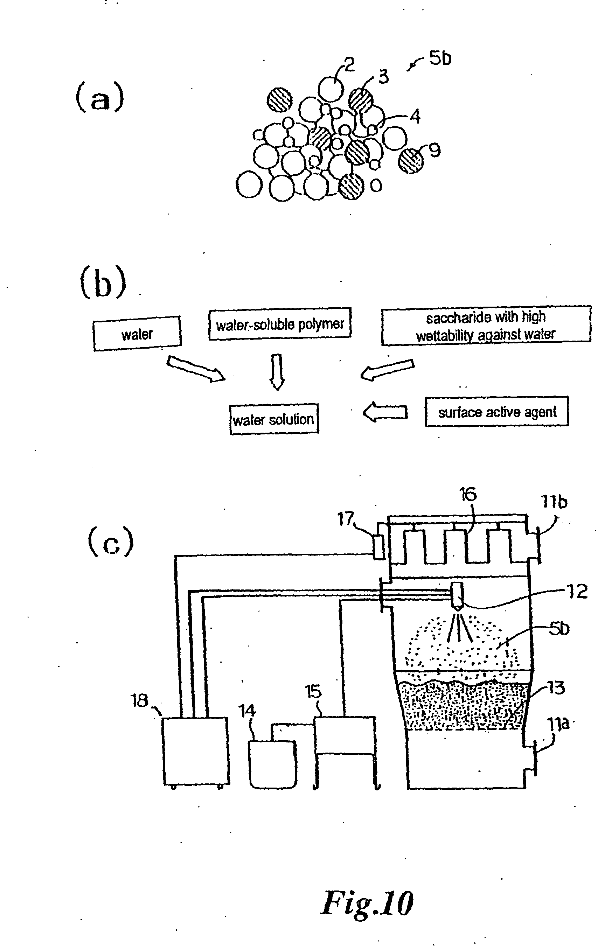

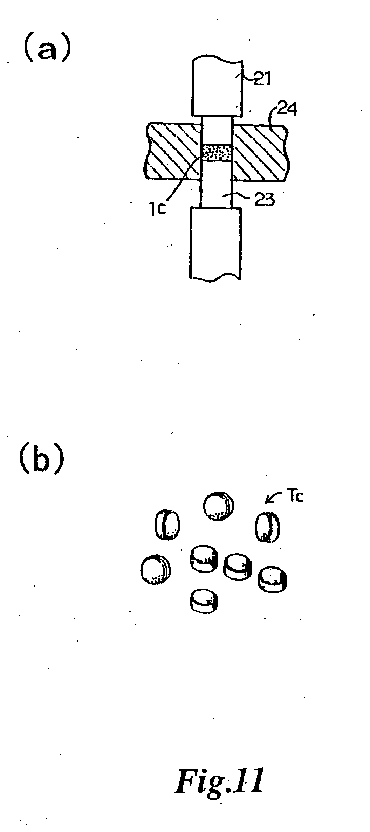

Fig.10 and Fig.11 are process drawings schematically showing another embodiment of a method of manufacturing the intrabuccally rapidly disintegrable tablet of the present invention.

The method of manufacture is the same as that described for manufacturing the tablet (intrabuccally rapidly disintegrable tablet) Tb as shown in Fig.6 and Fig.7.

More specifically, and according to this method of manufacture, a mixed powder of particles 5b is prepared by homogeneously mixing a principal agent particle 2, a saccharide particle 3 with high wettability against water, a disintegrant particle 4 ···, and a saccharide particle with high moldability 9 ··· with a mixer.

Then, an aqueous solution in which an appropriate amount of surface active agent is dissolved other than a water-soluble polymer and a saccharide with high wettability against water is prepared for granulating the mixed powder 5b in the process in Fig.10(b).

As shown in Fig.10(c), an aqueous solution in which a water-soluble polymer, a saccharide with high wettability against water, and a surface active agent are dissolved is prepared according to the procedure Fig.10 (b) and is sprayed from a spraying means 12 in order to granulate the mixed powder of particles 5b.

Then, as shown in Fig.11(a), the thus produced granulated material 1c is compressed and molded to produce the tablet Tc (intrabuccally rapidly disintegrable tablet) (See Fig.11(b)).

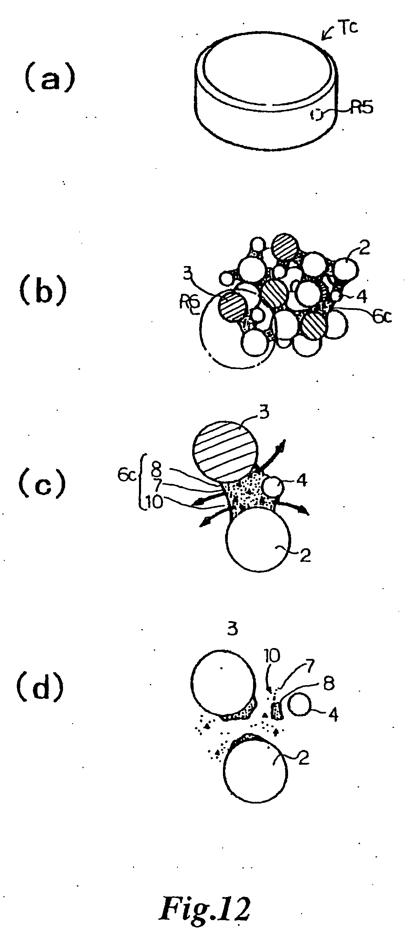

Fig.12 is an explanatory view diagrammatically showing how the tablet Tc (intrabuccally rapidly disintegrable tablet) is disintegrated in the mouth. Fig.12(a) is a perspective view of the intrabuccally rapidly disintegrable tablet Tc, Fig.12(b) is a diagrammatic view in which the area R5 in Fig.12(a) is enlarged, Fig.12(c) is a diagrammatic view in which the area R6 in Fig.12(b) is enlarged, and Fig.12(d) is a diagrammatic view showing how the tablet Tc (intrabuccally rapidly disintegrable tablet) is disintegrated in the saliva.

In this tablet Tc (intrabuccally rapidly disintegrable tablet), the binding between the particles 2 ···, 3 ···, 4 ···, 9 ··· of the granulated material 1c included in the tablet Tc is achieved by a water-soluble polymer 7 identical to that of the tablet Ta and Tb shown in Fig.12 (b) and Fig.12(c).

Therefore, when the tablet Tc is not in contact with water, the particles 2 ···, 3 ···, 4 ···, 9 ··· are bound by a water soluble polymer 7 so that each granulated material 1c is hard and its resistance to chipping is comparable to that of a conventional tablet. The tablet Tc is not chipped easily during storage and transportation.

Furthermore, the inclusion of a saccharide particle with high moldability 9 ··· in the granulated material 1c, decreases problems such as sticking, laminating, and capping during the process of compressing and tabletting the granulated material 1c.

Additionally and as shown in Fig.12(d), when the tablet Tc is inserted in the mouth, the tablet Tc (intrabuccally rapidly disintegrable tablet) rapidly disintegrates in the mouth through the same_action as with the tablets Ta, Tb because the saccharide particle (precipitation of fine particle) 8 with high wettability against water is dispersed in a binder 6 as in tablets Ta, Tb.

Furthermore, when the saccharide particles 3 with high wettability against water and the saccharide particles (precipitation of fine particle) 8 with high wettability against water are dissolved in saliva, its viscosity does not become higher than that of water $\eta w$. Thereby, when the saccharide particles 3 with high wettability against water and the saccharide particles (precipitation of fine particle) 8 with high wettability against water are dissolved in saliva, its viscosity remains such that it may quickly permeate the tablet Tc. The tablet Tc thereby rapidly disintegrates in the mouth.

Furthermore, in the tablet Tc, the particle 2 ···, 3 ···, 4 ···, 9 ··· are bound by a binder 6c that contains a surface active agent 10 ··· other than saccharide particles (precipitation of fine particle) 8 with high wettability against water.

Therefore, when the tablet Tc is inserted in the mouth, the binder 6c is easily moistened by water because the surface active agent 10 contained in the binder 6c decreases the surface tension of the water in the saliva. Then, the saccharide particles (precipitation of fine particle) 8 with high wettability against water is moistened and dissolved or dispersed in the saliva. The binding force between the particles 2 ···, 3 ···, 4 ···, 9 ··· in the binder 6c fails, so that the granulated material 1c is rapidly disintegrated.

The granulated material 1c is constituted of a mixed powder 5b comprising a principal agent particle 2, a saccharide particle 3 with high wettability against water, a disintegrant particle 4, and a saccharide particle with high moldability 9 bound by a binder 6c including a saccharide particle (precipitation of fine particle) 8 with high wettability against water and a surface active agent 10. The mixed powder of particles 5a including a principal agent particle 2, a saccharide particle 3 with high wettability against water, and a disintegrant particle 4 may be bound with a binder 6c including a saccharide particle (precipitation of fine particle) 8 with high wettability against water and a surface active agent 10. The granulated material may then be compressed so as to produce a tablet (intrabuccally rapidly disintegrable tablet).

The present invention is illustrated in further detail by the following non-limiting examples.

An example of preparation of a placebo tablet is first presented in order to facilitate the understanding of the following examples.

(Example producing a tablet by an internal lubricating method)

A mannitol powder (Japanese Pharmacopoeia) and a lactose powder (Japanese Pharmacopoeia) were prepared and filtered with a fixed coarse mesh, so as to produce a granular material with a desired particle diameter and particle distribution.

Then, the mannitol powder and the lactose powder with the desired particle diameter and particle distribution were combined so as to obtain a volume ratio of mannitol powder and lactose powder of 7 : 3. An appropriate amount of disintegrant was added (5 volume% of povidone on the entire volume was used ) and they were homogeneously mixed with a mixer to produce a mixed powder of particles.

A binder solution used for granulation was prepared comprising a volume of polyvinyl alcohol (PVA) equal to 1 volume% and a volume of mannitol equal to 5 volume%.

Then, the mixed powder of particles in which mannitol powder, lactose powder, and a disintegrant were homogeneously mixed was stored in a granulation tank of a fluid-layer granulation means and was blended with a heated air so as to form a fluidized layer. The binder solution which was prepared to include 1 volume% of polyvinyl alcohol (PVA) and 5 volume% of mannitol was sprayed onto the mixed powder by a spay provided in the granulation tank. The mixture was then dried to produce a granulated material with an average particle diameter of 250 μm.

Then, 0.5 weight% of magnesium stearate was added as a lubricant in the granulated material. The granulated material was then tabletted with an upper punch of 7mm diameter and a lower punch of 7mm diameter at a tabletting pressure of 133kg/punch by means of the rotary tabletting machine (Hata Tekkosho Co., Ltd.). A 100mg tablet (intrabuccally rapidly disintegrable tablet) having a diameter of 7mm and a thickness of 2.5mm was produced.

The hardness of the tablet was 3.9kg in average (the number of measured tablet n=3).

The disintegration time of the tablet in water was 15 seconds (the number of measured tablet n=3).

No tabletting problems (sticking, laminating, and capping) were observed.

(Example producing a tablet by an external lubricant spraying method)

A mannitol powder (Japanese Pharmacopoeia) and a lactose powder (Japanese Pharmacopoeia) were prepared and filtered with a fixed coarse mesh so as to produce a granular material with a desired particle diameter and particle distribution.

Then, the mannitol powder and lactose powder with desired particle diameters and particle distributions were combined so as to obtain a volume ratio of mannitol powder and lactose powder of 7 : 3. An appropriate amount of disintegrant was added (5 volume% of povidone was used on the entire volume) and they were homogeneously mixed with a mixer to produce a mixed powder of particles.

The binder solution used for granulation was prepared comprising a volume of polyvinyl alcohol (PVA) equal to 1 volume% and a volume of mannitol equal to 5 volume%.

Then, the external lubricant spraying method was followed as shown in Fig.13 - Fig.22. No lubricant was added directly in the granulated material.

Then, the granulated material was tabletted with an upper punch of 7mm diameter and a lower punch of 7mm diameter at a tabletting pressure of 133kg/punch with the rotary tabletting machine (Hata Tekkosho Co., Ltd.). A 100mg tablet (intrabuccally rapidly disintegrable tablet) of having' a diameter of 7mm and a thickness of 2.5mm was produced.

The hardness of the tablet was 5.7kg in average. It was found that the hardness was increased about 30% comparing with that produced with the internal lubricating method (the number of measured tablet n=3).

The disintegration time of the tablet in water was 11 seconds in average and it was found that the disintegration time was about 25% shorter than that of the tablet produced by the internal lubricating method (the number of measured tablet n=3).

No tabletting problems (sticking, laminating, and capping) were observed.

From the above-mentioned results, it was found that the tablet produced by an external lubridant spraying method has higher hardness and a quicker disintegration time than that of the tablet produced by an internal lubricating method.

The construction of the external lubricant spraying type tabletting machine 51 and methods of manufacturing tablets with it are hereafter presented.

(Explanation of an external lubricant spraying type tabletting machine used for producing the tablet of the present invention)

Fig.13 shows a diagrammatic entire construction of an external lubricant spraying type tabletting machine used for producing the tablet of the present invention.

The external lubricant spraying type tabletting machine 51 comprises a pulsating vibration air generation means 61 for generating positive pulsating vibration air, a discharge means (quantitative feeder) 71 for mixing and dispersing a lubricant (powder) in the positive pulsating vibration air generated from the pulsating vibration air generation means 61, a rotary type tabletting machine 81, a lubricant spray means 91, a suction means 101, and a control means 111 provided for controlling and managing the entire external lubricant spraying type tabletting means 51.

The rotary type tabletting machine 81 is provided with a rotary table 85 having plural dies 84 ···, plural upper punches 82 ···, and plural lower punches 83 ···.

The lubricant spray means 91 is fixedly positioned above the rotary table 85 of the rotary tabletting machine 81 and is provided for spraying a lubricant on each surface (lower surface) of the upper punches 82 ···, each surface (upper surface) of the lower punches 83 ···, and each surface (inner circumference) of the dies 84 ··· of the rotary-type tabletting machine 81.

The suction means 101 is provided for discharging extra lubricant sprayed in the lubricant spraying means 91.

The pulsating vibration air generation means 61 is provided with an air source 62 such as a blower and a pulsating vibration air conversion means 63 for converting compressed air generated by the air source 62 to pulsating vibration air.

In this embodiment, the air source 62 is connected with the pulsating vibration air generation means 63 via a conduit C3.

Further, a flow rate control means 64 for controlling the pressure and the flow amount of compressed air generated by the air source 62 into a fixed pressure and flow amount is provided in midstream of the conduit C3.

In this embodiment, an open-close valve such as an electromagnetic valve for opening and closing the conduit C3 is used as the flow rate control means 64.

In Fig.13 the member shown as 65 is a driving means such as a motor for driving a rotary cam (refer to a rotary cam 67 in Fig.15) comprising a rotary cam mechanism (refer to a rotary cam mechanism 66 in Fig. 15) for producing pulsating vibration air of the pulsating vibration air conversion means 63.

In the external lubricant spraying type tabletting machine 51, the pulsating vibration air generation means 61 and the lubricant spraying means 91 are connected by the conduit C1.

The discharge means (quantitative feeder) 71 is connected in midstream of the conduit C1.

More specifically, the pulsating vibration air conversion means 63 of the pulsating vibration air generation means 61 is connected to the quantitative feeder 71 via a conduit C1a.

Furthermore, the quantitative feeder 71 is connected to the lubricant spraying means 91 via a conduit C1b.

In the above-mentioned construction, the positive pulsating vibration air generated by driving the pulsating vibration air generation means 61 is supplied to the lubricant spraying means 91 via the conduit C1 (conduit C1a, C1b).

Then, a fixed amount of lubricant L is supplied from the discharge means (quantitative feeder) 71 to the positive pulsating vibration air flowing from the pulsating vibration air generation means 61 to the lubricant spraying means 91 in the conduit C1b so that the lubricant L is mixed and dispersed in the positive pulsating vibration air.

Thus, the lubricant L mixed and dispersed in the positive pulsating vibration air is supplied to the lubricant spraying means 91 and is sprayed on the surface of the upper punch 82, lower punch 83 and the die 84 contained in the lubricant spraying means 91.

For facilitating explanation, the conduit connecting the pulsating vibration air generation means 61 and the discharge means (quantitative feeder) 71 is called as C1a and the conduit connecting the discharge means (quantitative feeder) 71 and the lubricant spraying means 91 is called as C1b hereinafter.

The suction means 101 such as a blower is connected to the lubricant spraying means 91 via the conduit C2 and the extra lubricant among the lubricant L supplied in the lubricant spraying means 91 is suck to be removed from the lubricant spraying means 91.

The control means 111 is connected to the pulsating vibration air generation means 61 via a signal line L1 in such a manner that signals can be sent and received between the control means 111 and the pulsating vibration air generation means 61. The pulsating vibration air generation means 61 is designed to be operated following the command of the control means 111.

Particularly, the signal line L1 introduced from the control means 111 is diverged into two signal lines L1a, L1b. The signal line L1a is connected to the flow rate control means 64 and the signal line L1b is connected to the driving means 65 of the pulsating vibration air conversion means 63. According to such construction, when a command is delivered to the flow rate control means 64 from the control means 111, the flow rate control means 64 is designed to control the conduit C3 at a fixed opening degree. Further, when the command is delivered to the driving means 65 from the control means 111, the driving means 65 is designed to rotate the rotary cam 67 at a fixed rotary speed.

The control means 111 is connected to the suction means 101 via the signal line L2 so that the signals can be sent and received between the control means 111 and the pulsating vibration air generation means 61 and the pulsating vibration air generation means 61 is designed to be operated by the command from the control means 111. According to such construction, when a command is delivered to the suction means 101 from the control means 111, the suction means 101 is designed to be controlled at a fixed suction amount.

A light scattering type powder density measuring means 112 is provided in midstream of the conduit C2.

Particularly, the powder density measuring means 112 in this embodiment is provided with a laser beam irradiation equipment (not shown) for exposing a laser beam and a light receiving means (not shown).

The laser beam irradiation equipment (not shown) and the light receiving means (not shown) are opposed so as to sandwich a measuring member (not shown) provided at the conduit C2.

The measuring member (not shown) is made of a clear material such as glass or acrylate resin in order that the scattering light scattered by the particle of the lubricant L passing through the measuring member (not shown) is received in the light receiving means when the laser beam is exposed from the laser beam irradiation equipment (not shown) to the measuring member (not shown).

The information received in the light receiving means (not shown) is sent to the control means 111 and the density of the lubricant L flowing in the measuring member (not shown) is calculated according to the density analysis program based on the well-known Mie Scatter Theory which is stored in a memory of the control means 111 in advance. Then the calculated result is shown on a display (not shown) of the control means 111.

Herewith, an operator can control the spraying amount of the lubricant (powder) L sprayed from the lubricant spraying means 91 by controlling the driving amount of the suction means 101.

Next, the construction and operation of the external lubricant spraying type tabletting machine 51 are explained in more detail, particularly the rotary-type tabletting machine.

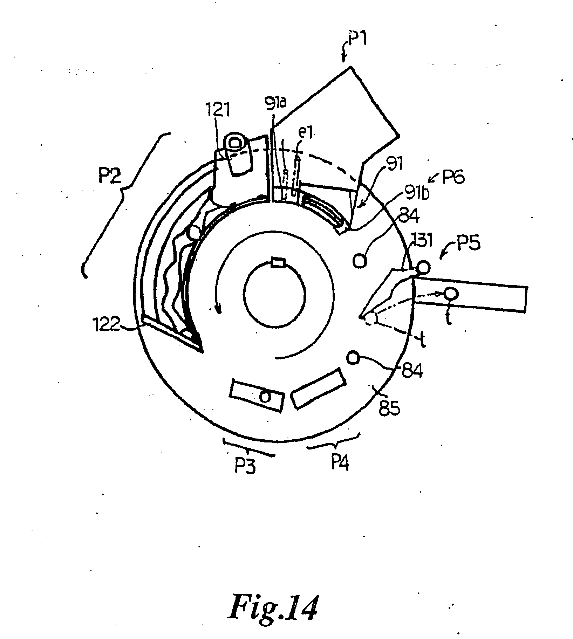

Fig. 14 is the plane view of the rotary-type tabletting machine 81 of the external lubricant spraying type tabletting machine 51 in Fig.13.

The rotary-type tabletting machine 81 is provided with a rotary table 85, numerous upper punches 82··· and lower punches 83 ···.

Numerous dies 84 ··· are formed along the circumference of the rotary table 85.

Each of the upper punches 82 ··· is provided so as to correspond to each of the dies 84 ··· formed on the rotary table 85. When the rotary table 85 is rotated at a fixed rotary speed, each upper punch 82 ··· is rotated at a fixed speed so as to synchronize with each corresponding die 84 ···.

As well, each lower punch 83 ··· is provided so as to correspond to each die 84 ··· provided on rotary table 85. When the rotary table 85 is rotated at a fixed speed, each lower punch 83 is rotated at a fixed speed so as to synchronize with each corresponding die 84 ···.

The numeral 86 shows a rotary cam mechanism used to execute rotary movement and vertical movement of the lower punches 83 . Practically each of the upper punches 82 ··· is also designed to execute rotary and vertical movements by a rotary cam mechanism for upper punches. However, for an easy understanding, the rotary cam mechanism for upper punches is not shown in the figure.

According to the rotary tabletting machine 81, the lubricant L is designed to be homogeneously applied on each surface of the inner circumference of the dies 84 ···, each surface of the lower surface of the upper punches 82 ··· and on each surface of the upper surface of the lower punches 83 ···, at a lubricant spray position P1 as shown in Fig.14.

More specifically, a lubricant spray means 91 is provided above the lubricant spray position P1 of the rotary table 85. Lubricant L is homogeneously applied on the surface of the inner circumference of the die 84, located above the upper surface of the lower punch 83, which is inserted in a fixed position in the die 84 when the die 84 is positioned in the lubricant spraying means 91, on the upper surface of the lower punch 83 and on the lower surface of the upper punch 82 according to rotation of the rotary table 85 while they are contained in the lubricant spray means 91.

The surface of the inner circumference of the die 84, the lower surface of the upper punch 82 and the upper surface of the lower punch 83, on which lubricant L is applied by the lubricant spray means 91 at the lubricant spray position P1, are sent to the molding material charge point P2. There the molding material is charged in a space formed by the upper surface of the lower punch 83 which is inserted at a fixed position in the die 84, and the inner circumference of the die 84.

More specifically, a molding material charge means (feed chute) 121 is fixedly provided above the molding material charge position P2 of the rotary table 85.

A molding material storage hopper (not shown) for storing molding material m is connected to the molding material charge means (feed chute) 121 via a material feed valve (not shown). The molding material m stored in the molding material storage hopper (not shown) is, or is not, supplied into the molding material charge means (feed chute) 121 by opening and closing the material feed valve (not shown).

When the material feed valve (not shown) is opened, molding material fed in the molding material charge means (feed chute) 121 is supplied from a zigzag groove of the molding material charge means (feed chute) 121 to the space formed by the die 84 and the lower punch 83 inserted at a fixed position in the die 84.

Extra molding material of the molding material m, supplied in the space formed by the die 84 and the lower punch 83 inserted at a fixed position in the die 84, is scraped and removed by a scraper 122 provided at a terminal end of the molding material charge means (feed chute) 121.

Following the above-mentioned procedures, the molding material m charged in the space formed by the die 84 and the upper surface of the lower punch 83 inserted at a fixed position in the die 84, is previously compressed with the upper punch 82 and the lower punch 83 corresponding to the die 84 at a pre-compression point P3, and then is compressed to produce a tablet t with the upper punch 82 and the lower punch 83 corresponding to the die 84 at an actual compression point P4.

When the die 84 comes to a material discharge point P5, the lower punch 83 inserted in the die 84 is raised to the upper end of the die 84. Then the tablet produced in the die 84 is discharged at a fixed position by a tablet discharge arm 131 that fixedly provided at the tablet discharge position P5 above the rotary table 85.

The position P6 in Fig.14 shows a cleaning position for cleaning each surface of the upper punch 82 ···, the lower punch 83 ···, and the die 84 ···. An upper punch cleaning means (not shown) , a lower punch cleaning means (not shown), and a die cleaning means (not shown) are provided at the cleaning position P6.

Next, construction and operation of the pulsating vibration air generation means 61 used.for the external lubricant spraying type tabletting machine 51 are explained in more detail.

Fig.15 is an explanatory view of the pulsating vibration air generation means 61.

As mentioned above, the pulsating vibration air generation means 61 is provided with the air source 62 and the pulsating vibration air conversion means 63 connected to the air source 62 via the conduit C3. The flow rate control means 64 is provided in midstream of the conduit C3.

The pulsating vibration air generation means 63 is provided with a valve chamber R having a valve seat 68 located between an input port 68a and an output port 68b, a valve plug 69 capable of opening and closing the valve seat 68, a cam mechanism for vertically moving the valve plug 69, and a case body 70 for containing the cam mechanism 66.

A penetrating hole 68h narrowing into the direction of the output port 68b is formed in the valve seat 68.

The valve plug 69 is narrowed like a cone so as to meet the shape of the penetrating hole 68h of the valve seat 68 so that the valve plug 69 can close the valve seat airtightly.

The valve plug 69 has an axis 69a which is contained in an axis hole 70h airtightly provided in a case body 70 and movable vertically.

A roller 69b is rotatably attached at the lower end of the axis 69a.

The cam mechanism 66 has a rotary cam 67 rotatably provided by means of a driving means such as a motor (a driving means 65 in Fig.13). The roller 69b rotatably attached at the lower end of the axis 69a of the valve plug 69 is rotatably held for the rotary cam 67.

Concavo-convex patterns p1, p2 are provided on the rotary cam 67.

More practically, the rotary cam 67 has an inner rotary cam 67a and an outer rotary cam 67b. The concavo-convex pattern p1 of the inner rotary cam 67a and the concavo-convex pattern p2 of the outer rotary cam 67b are aligned with each other allowing a space equivalent to a little larger than the diameter of the roller 69b.

The roller 69b is rotatably held between the inner rotary cam 67a and the outer rotary cam 67b. When the rotary cam 67 is rotated, the roller 69b accurately moves up and down according to the concavo-convex pattern p1 of the inner rotary cam 67a and the concavo-convex pattern p2 of the outer rotary cam 67b.

Accordingly, as the valve plug 69 accurately moves up and down according to the concavo-convex pattern p1 of the inner rotary cam 67a and the concavo-convex pattern p2 of the outer rotary cam 67b, the rotary plug 69 can open and close the penetrating hole 68h of the valve seat 68 according to the concavo-convex pattern p1 of the inner rotary cam 67a and the concavo-convex pattern p2 of the outer rotary cam 67b.

The input port 68a is connected to the air source 62 such as a blower, via the conduit C3.

In this embodiment, an example having the flow rate control means 64 in midstream of the conduit C3 is explained, however, the flow rate control means 64 is not always required.

The conduit C1 (more practically the conduit C1a) is connected to the output port 68b.

In Fig.15 the numeral 68c shows a flow rate control port.

An output control valve v is provided for the flow rate control port 68c.

The output control valve v is designed to adjust the flow rate control port 68c at a desired condition between a completely closed condition and a condition having a complete communication with the outer air.

The output control valve v is not limited if it can adjust the opening rate of the flow rate control port 68c at a desired rate so that conventional switch valves such as an electromagnetic valve may be used. However in the present invention, the flow rate control port 68c and the output control valve v are not always necessary.

A supply method of pulsating vibration air in the conduit C1 (more practically the conduit C1a) by means of the pulsating vibration air generation means 61 is explained hereinafter.

The rotary cam 67 having the concavo-convex patterns p1 and p2, can generate pulsating vibration air having wave shape suitable for the easy mixing of the lubricant. The rotary cam 67 is selected based on the particle diameter, particle distribution, and physical property of the lubricant particle contained in the storage hopper (the storage hopper 72 in Fig.13 and Fig.16) of the discharge means (quantitative feeder) 71.

Next, the thus selected rotary cam 67 is attached on a rotary axis 65a of the drive means (drive means 65 in Fig.13) for rotating the driving cam 67.

Then, if the flow rate control means 64 is provided, it is appropriately controlled.

If the output control valve v is provided, it is appropriately controlled.

The drive means (drive means 65 in Fig.13) is driven to rotate the rotary axis 65a at a fixed speed so that the rotary cam 67 is rotated at a fixed speed.

The air source 62 is driven, and compressed air with an almost constant air pressure and a fixed flow amount is supplied in the conduit C3.

The compressed air supplied in the conduit C3 is adjusted to the desired pressure and to the desired flow amount, and if the flow rate control means 64 is provided, is supplied to the valve chamber R.

The compressed air supplied in the valve chamber R is converted to pulsating vibration air by the rotary cam mechanism 66 provided in the casing 70, and is discharged to the conduit C1 from the output port 68b.

When the rotary cam 67 is rotated at a fixed speed, the valve plug 69 is moved up and down according to the concavo-convex patterns p1, p2 of the rotary cam 67. Thereby, the penetrating hole 68h of the valve seat 68 is controlled to be opened and closed by the valve plug 69, vertically moved by the concavo-convex patterns p1, p2 of the rotary cam 67, for example: fully open, half open, and fully closed.

The pulsating vibration air is discharged from the output port 68 to the conduit C1.

The pulsating vibration air generated from the pulsating vibration air generation means 61 is clearly different from the intermittent air generated by opening and closing a wave transmission pipe 4 in such a manner that a switching means such as an electromagnetic valve is provided for opening and closing the conduit C1, or the conduit C3, instead of the pulsating vibration air conversion means 63 in midstream of the conduit C1 or C3, connecting the air source 62 and the lubricant spray means 91.

This is explained hereinafter in more detail.

When a switching means such as an electromagnetic valve is provided for opening and closing the conduit C1 or the conduit C3 in midstream of the conduit C1 or C3, connecting the air source 62 and the lubricant spray means 91, intermittent air is simply fed in the conduit C1.

When the rotary cam 67 is rotated, the valve plug 69 vertically moves up and down according to the concave-convex patterns p1 and p2 of the rotary cam 67. Hence the penetrating hole 68h of the valve seat 68 is controlled to be opened and closed by the valve plug 69 vertically moving according to the concavo-convex patterns p1 and p2 of the rotary cam 67. Therefore, pulsating vibration air having wave shape according to the concavo-convex pattern p1 and p2 of the rotary cam 67 can be fed to the conduit C1.

When the drive amount control and flow rate control means 64 of the air source 62 is provided, and the adjustment and output control valve v of the flow rate control means 64 is provided, several kinds of pulsating vibration air having different frequency, amplitude, and wave shapes can be generated in the conduit C1 by controlling the output control valve v, the rotary speed of the rotary cam 67, and the exchange of the rotary cam 67 separately or in combination.

By such a control, a pulsating vibration air with a fixed frequency (period), having peaks and valleys of positive amplitudes, and a pulsating vibration air with a fixed frequency (period) having peaks of positive amplitude and valleys of almost atmospheric amplitude, can be produced.

The construction and operation of the discharge means (quantitative feeder) 71 used for the external lubricant spraying type tabletting machine 51 are detailed hereinafter.

Fig. 16 is a diagrammatic sectional view of the discharge means (quantitative feeder) 71.

The discharge means (quantitative feeder) 71 has a storage hopper 72, a cylindrical body 74 airtightly attached under a material discharge port 72a of the storage hopper 72, an elastic membrane 73 provided so as to close the bottom of the cylindrical body 74, and a dispersion chamber 75 connected to the material discharge port 72a of the storage hopper 72 via the elastic membrane 73.

The storage hopper 72 is provided so as to contain lubricant (powder) L.

Fig.17 is a plane view diagrammatically showing the elastic membrane.

An aperture (slit) 73s with a fixed length is provided at the center of the elastic membrane 73 so as to permit penetration of the membrane 73 as shown in Fig. 17

. In this embodiment, a fixed amount of lubricant L is always stored on the elastic membrane 73.

It is explained in more detail hereinafter.

The cylindrical body 74 is airtightly connected to the discharge port 72a of the storage hopper 72 as mentioned, and the elastic membrane is provided so as to construct the bottom of the cylindrical body 74.

A material feed valve 77 for opening and closing the material discharge port 72a of the storage hopper 72 is provided at an upper cylindrical body 74a of the cylindrical body 74.

In this embodiment, a lower cylindrical body 74b of the cylindrical body 74 is made of clear material such as polycarbonate, acrylate resin, or glass. A level sensor 76 is provided at a fixed height H of the lower cylindrical body 74b, above where the elastic membrane 73 is provided.

Each material feed valve 75 and level sensor 76 is connected to the control means (control means 111 in Fig.13) via a signal line (not shown) so that the material feed valve 75 opens or closes the material discharge port 72a of the storage hopper 72 based on the signals detected by the level sensor 76.

This embodiment uses a level sensor 76 having a light emitting element 76a and a light receiving element 76b.

Each light emitting element 76a and light receiving element 76b is designed to be attached on a support shaft P, P capable of adjusting its height.

The light emitting element 76a can emit visible light such as red light, or light such as infra-red radiation (hereinafter only called "light").

The light emitting element 76a and the light receiving element 76b are opposed so as to sandwich the lower cylindrical body 74b of the cylindrical body 74. Light emitted from the light emitting element 76a passes through the lower body 74b and is received at the light receiving element 76b.

In such a constructed discharge means (quantitative feeder) 71, when the amount of lubricant L on the elastic membrane 73 is lower than the height H, light emitted from the light emitting element 76a is received at the light receiving element 76b. However, when the amount of lubricant L on the elastic membrane 73 reaches the height H, the light emitted from the light emitting element 76a is blocked by the lubricant L so that the height H of the lubricant L on the elastic membrane 73, namely the amount of lubricant L on the membrane 73, can be detected.

In this discharge means (quantitative feeder) 71, the control means 111 controls in such a way that the material feed valve 77 is opened when the light receiving element 76b of the level sensor 76 receives light emitted from the light emitting element 76a and the material feed valve 77 is closed when the light receiving element 76b does not receive light emitted from the light emitting element 76a. Therefore, the amount of lubricant L on the elastic membrane 73 can be always kept at the height H above the elastic membrane 73.

If a level sensor 76 used has a light emitting element 76a which emits visible light such as red light, it is preferable that the lower cylindrical body 74b of the cylindrical body 74 be made of clear material having high translucency. Furthermore, it is preferable that the lower cylindrical body 74b be made of a material preventing lubricant L from attaching on the inner circumferential wall of the body 74b in order to accurately measure the amount of lubricant L in the body 74b. In light of this, it is preferable that the lower cylindrical body 74b be made of polycarbonate and its inner circumferential wall be mirror finish.

The dispersion chamber 75 is airtightly connected under the material discharge port 72a of the storage hopper 72 via the elastic membrane 73 as shown in Fig.16.

The dispersion chamber 75 is formed like a cylindrical tube, an introduction port 75a of the pulsating vibration air is provided at the lower part of the chamber 75, and a discharge port 75b of pulsating vibration air is provided above the introduction port 75a.

In this embodiment, the introduction port 75a is installed at the dispersion chamber so as to be parallel to a tangent of the inner circumference of the dispersion chamber 75. The discharge port 75b is also installed at the dispersion chamber 75 so as to be parallel to the tangent of the inner circumference of the dispersion chamber 75. Further, the introduction port 75a and the discharge port 75b are positioned so as to not oppose each other.

The conduit C1a is connected to the introduction port 75a and pulsating vibration air generated by the pulsating vibration air generation means 61 is supplied in the dispersion chamber 75 from the introduction port 75a.

The conduit (the conduit C1b in Fig.13) is connected to the discharge port 75b and pulsating vibration air supplied in the dispersion chamber 75 from the introduction port 75a is supplied to the conduit C1b.

In this embodiment and as mentioned above, the introduction port 75a is provided at the lower part of the chamber 75 tangentially adjusted on the inner circumference in the dispersion chamber 75. And the discharge port 75b is provided at the upper part of the chamber 75 tangentially adjusted on the inner circumference in the dispersion chamber 75.

By such a construction, the pulsating vibration air supplied in the dispersion chamber 75 from the introduction port 75a, becomes a swirling flow of pulsating vibration air from bottom to top, toward the discharge port 75b from the introduction port 75a in the dispersion chamber 75, so that the pulsating vibration air is discharged out of the dispersion chamber 75.

The pulsating vibration air swirling in the dispersion chamber 75 does not lose its feature.

The operation of the discharge means (quantitative feeder) 71 is explained hereinafter.

The lubricant L is contained in the storage hopper 72 when the discharge means (quantitative hopper) 71 is first operated. Then, the control means 111 is turned on.

Light is emitted from the light emitting element 76a of the level sensor 76.

While the light is received in the light receiving element 76b, the material feed valve 75 is opened, the lubricant L stored in the storage hopper 72 falls in the cylindrical body 74 to be accumulated on the elastic membrane 73.

When the amount of lubricant L accumulated on the elastic membrane 73 reaches height H above the elastic membrane 73, the light emitted from the light emitting element 76a is blocked by the lubricant L accumulated on the elastic membrane 73. When the light receiving element 76b does not receive light, such as red light or infra-red radiation, emitted from the light emitting element 76a, the material feed valve 75 remains closed.

The amount of lubricant L on the elastic membrane 73 is thereby kept at height H above the membrane 73.

While the discharge means (quantitative feeder) 71 is operated, the light emitting element 76a of the level sensor 76 is always turned on.

Therefore, the amount of lubricant L on the elastic membrane 73 is kept at height H above the elastic membrane while operating the discharge means (quantitative feeder) 71.

Then, the pulsating vibration air generation means 61 is driven, and generated pulsating vibration air is supplied in the conduit C1a.

The pulsating vibration air supplied in the conduit C1a transfers in the conduit C1a and is supplied in the dispersion chamber 75 from the introduction port 75a thereof.

Fig.18 is a view explaining the operation of the elastic membrane 73 comprising the discharge means (quantitative feeder) 71.

As mentioned above, the pulsating vibration air swirling in the dispersion chamber 75 does not lose its feature. Therefore, when the amplitude of the pulsating vibration air peaks, the pressure in the dispersion chamber 75 is heightened because of the property of the pulsating vibration air so that the elastic membrane 73 is elastically deformed and its center is curved upward as shown in Fig.18(a).

The aperture (slit) 73s is then shaped like a letter V of which the upper part is opened as shown in Fig.

18(a). Then, a part of the lubricant L on the elastic membrane 73 in the cylindrical body 74 falls in the V-shaped aperture (slit) 73s.

When the pulsating vibration air supplied in the dispersion chamber 75 changes from a peak to a valley and the pressure in the chamber 75 is lowered, the elastic membrane 73 curved upward is elastically deformed so as to be returned to its original shape because of resilience as shown in Fig.18(b).

Because the V-shaped aperture (slit) 73s is going to return to its original closed condition, the lubricant L fallen in the aperture (slit) 73s is sandwiched therein.

Then the amplitude of the pulsating vibration air supplied in the dispersion chamber 75 passes to the valley, the pressure in the dispersion chamber 75 is further lowered, and thus the elastic membrane 73 at its original condition is elastically deformed corresponding to its resilience force and/or lowering of the pressure in the dispersion chamber 75 so that its center is curved downward as shown in Fig.18(c).

The aperture (slit) 73s is formed like a reversed letter V of which the bottom is opened as shown in Fig. 18(c). The lubricant L sandwiched in the aperture (slit) 73s falls in the dispersion chamber 75 so that the lubricant L is mixed with the pulsating vibration air swirling, dispersed in the dispersion chamber 75, and then fed to the conduit C1b from the discharge port 75b.

The above-mentioned operations are repeated while the discharge means (quantitative feeder) 71 is operated.

The lubricant L on the elastic membrane 73 is reduced by discharging lubricant L into the dispersion chamber 75 from the aperture (slit) 73s because of the vibration of the elastic membrane 73 as shown in Fig. 18(a) - Fig.18(c). When the amount of the lubricant L on the elastic membrane 73 is reduced from height H, at which the level sensor 76 is provided above the elastic membrane 73, the light receiving element 76b of the level sensor 76 receives light emitted from the light emitting element 76a.

In this embodiment the control means 111 opens the material feed valve 77 until the light receiving element 76b stops receiving light emitted from the light emitting element 76a. Therefore, while the discharge means (quantitative feeder) 71 is operated, a fixed amount of lubricant L is always present on the elastic membrane 73. There is no phenomenon that permits the amount of lubricant L discharged in the dispersion chamber 75 from the aperture (slit) 73s, to vary because of a variation in the amount of lubricant L on the elastic membrane 73.

Furthermore, the vibration of the elastic membrane 73 as shown in Fig.18(a) - Fig.18(c) depends on the frequency, period, amplitude and wave shape of the pulsating vibration air supplied in the dispersion chamber 75. Therefore, if the pulsating vibration air is constant, a fixed amount of lubricant L can always be accurately mixed with a fixed amount of air.

When the pulsating vibration air is made constant, the amount of lubricant L mixed and dispersed in a fixed amount of air can be varied if the size (length) of the aperture (slit) 73s of the elastic membrane 73 is changed.

If the size (length) of the aperture (slit) 73s of the elastic membrane 73 is not changed, at least one of the frequency, period, amplitude and wave shape of the pulsating vibration air is changed, and the amount of lubricant L mixed and dispersed in a fixed amount of air can be varied.

In this discharge means (quantitative feeder) 71, the vibration of the elastic membrane 73 is determined according to the frequency, period, amplitude and wave shape of the pulsating vibration air. As a result, a fixed amount of lubricant L while dispersing in air can always be fed in the conduit C1b, by simply controlling the frequency, period, amplitude and wave shape of the pulsating vibration air.

Furthermore, in this discharge means (quantitative feeder) 71, the pulsating vibration air is made by a swirling flow directed from the introduction port 75a to the discharge port 75b in the dispersion chamber 75 as mentioned above, so that the lubricant L fallen in the dispersion chamber 75 is sucked in the swirling pulsating vibration air and fed to the conduit C1b after a large lubricant powder L is broken into a desired particle diameter.

This is detailed hereinafter.

In this discharge means (quantitative feeder) 71, pulsating vibration air is swirled upwardly from the introduction port 75a to the discharge port 75b as mentioned above, so that the dispersion chamber 75 performs size classification like a cyclone does. Thereby, even if there are large particles in the lubricant L fallen in the dispersion chamber 75, these particles keep swirling at the lower part of the chamber 75 so that they are not fed out to the conduit C1b from the discharge port 75b.

Furthermore, when large particles keep swirling at the lower part of the dispersion chamber 75, they are broken into desired particle diameters by the pulsating vibration air. The broken particles then ride on the upwardly swirling pulsating vibration air from the introduction port 75a to the discharge port 75b and are fed to the conduit (conduit C1b in Fig.13) which is connected to the discharge port 75b.

Therefore, when the lubricant L is mixed and dispersed in the pulsating vibration air by means of the discharge means (quantitative feeder) 71, a fixed amount of lubricant L having a fixed particle size is always supplied to the lubricant spraying means (lubricant spraying means 91 in Fig.13). As a result, the lubricant L can be homogeneously applied on each upper punch 82 ···, lower punch 83 ···, and die 84 ···, without applying large particle of the lubricant powder L.'

Even if a large particle is included in the lubricant powder L supplied in the dispersion chamber 75, almost all of it is broken into a fixed particle size and is supplied to the lubricant spray means 91 so that hardly any large particles of lubricant L accumulate in the dispersing chamber 75.

When such an external lubricant spraying type tabletting machine 51 is used during continuous compression to produce tablets t, no interruption of tabletting operation is required , which would otherwise have been necessary in order to clean accumulated large particles of lubricant L in the dispersion chamber 75.

The numeral 78 in Fig.16 designates a remaining lubricant detection sensor for detecting the remaining amount of lubricant L stored in the storage hopper 72. The detection sensor 78 has a light emitting element 78a for emitting visible light such as red light or light such as infra-red radiation, and a light receiving element 78b for receiving the light emitted from the light emitting element 78a. The light emitting element 78a and the light receiving element 78b oppose each other at a fixed position above the material discharge port 72a of the storage hopper 72.

The remaining amount detection sensor 78 is connected to the control means (control means 111 in Fig.13) via a signal line (not shown).

The light emitting element 78a of the detection sensor 78 is always turned on while the discharge means (quantitative feeder) 71 is operated.

When an adequate amount of lubricant L is stored in the storage hopper 72, the light emitted from the light emitting element 78a is blocked by the lubricant L so that the light receiving element 78b does not receive light such as red light or infra-red radiation emitted from the light emitting element 78a.

The amount of lubricant L stored in the storage hopper 72 is reduced to a point where it is lower than the position where the light emitting element 78a and the light receiving element 78b are provided. The light emitted from the light emitting element 78a is then received by the light receiving element 78b.

In this embodiment, when the light receiving element 78b of the detection sensor 78 receives the light emitted from the light emitting element 78a, a display means (not shown) of the control means 111 shows that the amount of the lubricant L in the storage hopper 72 is reduced so as to inform an operator that supply of lubricant L is needed in the storage hopper 72.

The methods for so informing the operator according to the present invention are not limited. For instance, an alarm lamp may be turned on or a warning buzzer may be sounded. In this embodiment only preferable embodiments are exemplified and the lubricant remaining amount detection sensor 78 is not always necessary.

The numeral 79 in Fig. 16 shows an observation means for viewing the condition of the lubricant L discharged in the cylindrical body 74 from the storage hopper 72.

The observation means 79 is connected to the control means (control means 111 in Fig.13) via a signal line (not shown).

The observation means 79 has an electronic flash 79a and an imaging means 79b such as a solid-state imaging means like a liquid-crystal camera.

In this embodiment the control means 111 turns on the electronic flash 79a periodically or if required, and the condition of lubricant L discharged in the cylindrical body 74 from the storage hopper 72 is taken by the imaging means 79b. The image information taken by the imaging means 79b is temporally expanded and stored in an image memory of the control means 111 and is analyzed by an image analysis program stored in the memory means of the control means 111 in advance. When the lubricant L is not smoothly discharged in the cylindrical body 74 from the storage hopper 72, such information is displayed on the display means (not shown) of the control means 111 to inform an operator.

Observation means 79 having an electronic flash 79a and an imaging means 79b are explained above by way of examples only. For example, an observation means may be used having a laser beam emitting means for emitting laser beam and a light receiving means for receiving scattering beam emitted by the laser beam emitting means. In this embodiment, the laser beam is scattered by the lubricant particles L falling in the cylindrical body 74 from the storage hopper 72 and the condition of the lubricant L discharged in the cylindrical body 74 from the storage hopper 72 is analyzed with on the well-known Mie Scattering Theory. The observation means 79 is not always required and is only described by way of a preferred embodiment.

The numeral 80 in Fig. 18 shows an observation means for viewing the condition of the lubricant L in the dispersion chamber 75.

The observation means 80 is also connected to the control means (control means 111 in Fig.13) via a signal line (not shown).

This embodiment uses a probe type light scattering observation means incorporating an integrated combination of a laser beam emitting means for emitting laser beam, and a light receiving means provided apart at a fixed distance from the laser beam emitting means.

When the probe type observation means 80 is inserted in the dispersion chamber 75, a scattering light is emitted from the laser beam emitting means, is scattered by the lubricant particles L passing between the laser beam emitting means and the receiving means and is received in the light receiving means. The information received by the light receiving means is then processed by a program based on a well-known Mie Scattering Theory stored in the control means 111 to analyze the condition of lubricant L in the dispersion chamber 75. In case of an abnormality in the condition of lubricant L in the dispersion chamber 75, the abnormality is shown on the display (not shown) of the control means 111 so as to call an operator's attention.

The construction and operation of the lubricant spraying means 91 used for the external lubricant spraying type tabletting machine 51 are explained in detail below.

Fig.19 is a plane view diagrammatically showing the construction of a lubricant spraying means 91.

The lubricant spraying means 91 has a lower punch lubrication means 92 and an upper punch lubrication means 93. Both of them are fixedly provided at a fixed position above the rotary table 85 of the rotary type tabletting machine (rotary tabletting machine 81 in Fig. 13).

The numeral 94 in Fig.19 is a fixing table for fixedly positioning the lubricant spraying means 91.

Fig.20 is an outer perspective view diagrammatically showing the upper punch lubrication means 93 of the lubricant spraying means 91 in Fig.19, when seen from the periphery of the rotary table 85 into the center thereof.

Fig.21 shows a diagrammatic section along the line I-I in Fig.19. Fig.22 shows a diagrammatic section along the line II-II in Fig.19.

In this embodiment, the upper punch lubrication means 93 is positioned forward (upper stream) of the rotational direction of the rotary table 85 against the lower punch lubrication means 92.

The rotary table 85 is rotated under the lubricant spraying means 91 in such a manner that its surface S85 is almost touched at a lower surface S92b of the lower punch lubrication means 92 of the lubricant spraying means 91, and at a lower surface S93b of the upper punch lubrication means 93.

A guide groove g1 is formed on an upper surface S92a of the lower punch lubrication means 92 and comprises each passage of the upper punches 82 ··· along the rotary orbit of the upper punches 82 ··· in such a manner that each one of the numerous upper punches 82 ···, synchronously rotating with the rotary table 85, sequentially passes in the guide groove g1. When the position of the upper surface S92a of the lower punch lubrication means 92 is lower than the lower surface of each of the numerous upper punches 82 ···, synchronously rotating with the rotary table 85, such a guide groove g1 is not required.

The lower punch lubrication means 92 is provided in a circumferential direction of the rotary table 85 so as to cover the numerous dies 85 ··· from above, the dies being sequentially fed under the lower punch lubrication means 92 by rotation of the rotary table 85.

. The lower surface S92b of the lower punch lubrication means 92 is smoothly surfaced so as to smoothly slide the rotary table 85.

The numeral e1 in Fig.19 and Fig.21 shows a lubricant introduction pipe and a conduit. The conduit (conduit C1b in Fig.13) is connected to the lubricant introduction pipe e1.

The lubricant (powder) L fed while dispersing in the pulsating vibration air from the lubricant introduction port e1 to the lower punch lubrication means 92 via the conduit C1b, is designed to be discharged from a discharge port e2 provided at the lower surface side S92b of the lower punch lubrication means 92 via a penetrating passage h1. The lubricant (powder) L fed while being dispersed in the pulsating vibration air and discharged from the discharge port e2 is sequentially sprayed on each die 84 ··· fend under the lower punch lubrication means 92, and each lower punch 83 ··· inserted into a fixed position in each die 84 ···.

In more detail, the discharge port e2 vertically faces the upper surface of the lower punch 83 inserted at a fixed position in the die 84 which is positioned under the lower punch lubrication means 92. Because of the aspect of the discharge port e2, the lubricant (powder) L fed while being dispersed in the pulsating vibration air and discharged from the discharge port e2 of the lower punch lubrication means 92, is designed to be sprayed almost vertically on the upper surface of the lower punch 83 which is inserted in the die 84 fed below the lower punch lubrication means 92 by rotation of the table 85. Therefore, the lubricant (powder) L is applied on the upper surface of the lower punch 83 and the upper part of the circumferential wall of the die 84 above the lower punch 83.

A long groove c1 is formed on the lower surface S92b of the lower punch lubrication means 92 towards the upper punch lubrication means 93 from the position of the discharge port e2.

Lubricant (powder) L tends to be attached excessively on the upper surface S83 of the lower punch 83 under gravity.

However, even if the extra lubricant (powder) is attached on the upper surface S83 of the lower punch 83, such lubricant is blown out when the pulsating vibration air blown together with the lubricant L is at its peak so that the extra lubricant does not attach on the upper surface S83 of the lower punch 83.

Further, the lubricant (powder) L blown from the upper surface S83 of the lower punch 83 is attached on the inner circumference S84 of the die 84 so that lubricant can be homogeneously attached on the inner circumference of the die 84 above the upper punch 83.

A long groove c2 is formed on the lower surface S93b of the upper punch lubrication means 93 along the rotary orbit of the numerous dies 84 ··· provided for the rotary table 85.

The long groove c2 is connected with the long groove c1 provided on the lower surface S92b of the lower punch lubrication means 92.

An upper punch containing groove g2 is formed above the long groove c2 of the upper punch lubrication means 93 for sequentially containing each rotating upper punches 82 ···along the rotary orbit of the upper punch 82 ··· in such a manner that each one of the numerous upper punches 82 ··· rotates synchronously with the rotary table 85 sequentially passing in the groove g2.

Further, a slit 93s is provided at the center of the bottom of the upper punch containing groove g2 so as to penetrate therethrough along the rotary orbit of the upper punches 82 ···.

. A suction head 95 is provided above the upper punch containing groove g2, and the conduit C2 connected to the suction means 101 in Fig.13 is connected to the suction head 95.

A suction port 95h of the suction head 95 is provided along the upper punch containing groove g2 from a tip end es to a terminal end ee.

According to the shape of the suction port 95h of the suction head 95, when the suction means (suction means 101 in Fig.13) is driven, air flow (negative flow) from the slit 93s to the suction port 95h is homogeneously generated between the tip end es to the terminal end ee of the slit 93s above the upper punch containing groove g2 of the upper punch lubrication means 93.

Air flow directed to the long groove c2 of the upper punch lubrication means 93 from the lower punch lubrication means 92 is generated in a space between the long groove c1 formed on the lower surface S92b of the lower punch lubrication means 92 and the surface S85 of the rotary table 85.

Air flow (negative flow) homogeneously directed from the end of the long groove c1 to the slit 93s is generated between the end of the long groove c1 and the end ee from the tip end es of the slit 93s in a space formed by the long groove c2 of the upper punch lubrication means 93 and the surface S85 of the rotary table 85.

The extra amount of lubricant (powder) L blown on the surface (inner circumference) of the die 84 fed under the lubricant spraying means 91, the surface (upper surface) of the lower punch 83 inserted in the die 84 together with the pulsating vibration air is moved into the long groove c2 of the upper punch lubrication means 93 by an air flow (negative flow) generated by the suction means 103 from the lower punch lubrication means 92 to the long groove c2 of the upper punch lubrication means 93 in a space formed by the long groove c1 and the surface S85 of the rotary table 85 and by cooperation of this air flow (negative flow) and pulsating vibration air. Then the lubricant is homogeneously moved to the suction port 95h from between the tip end es and the end ee of the slit 93s.

The air flow (negative flow) moving into the suction port 95h above the slit 93s is substantially laminar air flow in substance. In order to achieve this, a curb plate type current plate member may be provided on the slit 93s so as not to touch each surface (lower surface) of the upper punches 82 ··· which passes in the upper punch containing groove g2 of the upper punch lubrication means 93.

The lubricant (powder) L keeps colliding for a long time with the surface (lower surface) of each upper punch 82 ··· passing in the upper punch containing groove g2 of the upper punch lubrication means 93 under substantial laminar air flow (negative flow), while each upper punch 82 ··· moves from the tip end es to the end ee of the slit 93s. Therefore, the lubricant (powder) L can be sequentially attached on the surface (lower surface) of each upper punch 82 ···, on which surface the lubricant (powder) L is hardly attached because of the gravity.

The extra amount of lubricant (powder) L supplied under substantial laminar air flow (negative flow) into the surface (lower surface) of each upper punch 82 ··· and lubricant (powder) L excessively attached on the surface of each upper punch 82 ···, is discharged in the suction port 95h by the substantial laminar air flow (negative flow). As a result, lubricant (powder) L is homogeneously applied on the surface (lower surface) of each upper punch 82 ··· in just proportion.

The numeral e3 in Fig.19 and Fig.21 shows a connection port for connecting the conduit (conduit C2 in Fig.13).

The numeral h2 in Fig.19 and Fig.21 shows a long suction port provided for the lower surface S92b of the lower punch lubrication means 92 so as to direct to the center from the periphery of the rotary table 85. The numeral e4 shows a connection port for connecting a conduit (not shown) connected to a suction means (not shown) to the suction port h2.

The suction port h2 is provided so as to remove extra lubricant (powder) L from the inner circumference S84 of the die 84 and the upper surface S83 of the lower punch 83, on which lubricant (powder) L is applied, and to remove unnecessary lubricant (powder) L attached around the dies 84 of the rotary table 85 from the discharge port e2 of the lower punch lubrication means 92.

In this embodiment, the upper punch lubrication means 92 having a suction port h2 is explained, however, the suction port h2 is not always necessary for the external lubricant spraying type tabletting machine 51.

Next, the process of applying lubricant (powder) L on the surface (lower surface) of each upper punch 82 ···, the surface (upper surface) of each lower punch 83 ···, and the surface (inner circumference) of each die 84 ··· is explained hereinafter.

The control means 111 is turned on.

A main power of the rotary type tabletting machine 81 is turned on and a main power of the pulsating vibration air generation means 61 is also turned on.

Depending on the physical property and chemical nature of the used lubricant (powder), the rotary cam 67 having suitable concavo-convex patterns p1, p2 for generating the pulsating vibration air in which lubricant (powder) is easily mixed and dispersed, is attached on the rotary axis 65a of the drive means 65 of the pulsating vibration air conversion means 63 of the pulsating vibration air generation means 61.

The granulated material produced as mentioned above is contained as molding material in the molding material storage hopper (not shown) of the molding material charge means (feed chute) 121.

The lubricant (powder) L is contained in the storage hopper 72 of the discharge means (quantitative feeder) 71

The level sensor 76 of the discharge means (quantitative feeder) is turned on so as to make the material feed valve 75 active.

When the level sensor 76 is turned on, the material feed valve 75 is opened so that the lubricant (powder) L is discharged from the storage hopper 72 into the cylindrical body 74.

When the amount of the lubricant (powder) L in the cylindrical body 74 reaches height H above the elastic membrane 73 of the cylindrical body 74 of the discharge means (quantitative feeder) 71, the material feed valve 75 closes and the discharge of the lubricant (powder) L in the cylindrical body 74 from the storage hopper 72 is stopped.

Then the suction means 101 and the rotary type tabletting machine 81 are driven.

Accordingly, the numerous upper punches 82 ···, lower punches 83 ···, and the rotary table 85 of the rotary type tabletting machine 81 are synchronously rotated at a fixed speed.

Also the air source 62 of the pulsating vibration air generation means 61 and the drive means 65 are driven so as to rotate the rotary cam 67 at a fixed speed.

If the flow rate control means 64 is provided, it may be properly adjusted.

If the output control valve v is provided, it may be properly adjusted.

The drive means 65 is driven and the rotary cam 67 is controlled so as to rotate at a fixed speed by means of the control means 111.

The drive amount of the air source 62 is also controlled if required.

As mentioned above, desired pulsating vibration air is fed in the conduit C1b.

The pulsating vibration air fed in the conduit C1b then enters into the dispersion chamber 75 from the introduction port 75a and becomes a swirling flow from the introduction port 75a toward the discharge port 75b.

Herewith, the elastic membrane 73 vibrates according to the period, frequency, amplitude and wave shape of the pulsating vibration air. When the aperture (slit) 73s of the elastic membrane 73 is opened and closed, then the lubricant (powder) L on the elastic membrane 73 in the cylindrical body 74 is dropped.

The lubricant (powder) L thus fallen in the dispersion chamber 75 is sucked in the swirling pulsating vibration air, mixed and dispersed therein.

The lubricant (powder) L thus mixed and dispersed in the pulsating vibration air is fed to the conduit C1b from the discharge port 75b of the dispersion chamber 75.

The pulsating vibration air fed in the conduit C1b is supplied in the lower punch lubrication means 92 from the lubricant introduction port e1.

The lubricant (powder) L thus fed in the lower punch lubrication means 92 from the lubricant introduction port e1 is sprayed on the die 84, fed under the lubricant spraying means 91 according to rotation of the rotary table 85 and the lower punch 83 inserted at a fixed position of the die 84 from the discharge port e2 of the lower punch lubrication means 92, together with pulsating vibration air.

The extra amount of lubricant (powder) L sprayed on the die 84, fed under the lubricant spraying means 91 and the lower punch 83 inserted at a fixed position of the die 84 together with pulsating vibration air, moves in the long groove c2 of the upper punch lubrication means 93 and homogeneously moves into the suction port 95h from between the tip es and the end ee of the slit 93s by air flow (negative flow) directed to the long groove c2 of the upper punch lubrication means 93 from the lower punch lubrication means 92 in the space formed by the long groove c1 and the surface S85 of the rotary table 85, and by cooperation of this air flow (negative flow) and the pulsating vibration air when the suction means (suction means 101 in Fig.13) is driven.

The lubricant (powder) L keeps colliding for a long time with the surface (lower surface) of each upper punch 82 ···, passing in the upper punch containing groove g2 of the upper punch lubrication means 93 under substantial laminar air flow (negative flow), while each upper punch 82 ··· moves from the tip end es to the end ee of the slit 93s. Therefore, the lubricant (powder) L can be sequentially attached on the surface (lower surface) of each upper punch 82 ··· on which surface, the lubricant (powder) L is hardly attached because of gravity.

The extra amount of lubricant (powder) L supplied under substantial laminar air flow (negative flow) into the surface (lower surface) of each upper punch 82 ··· and the lubricant (powder) L excessively attached on the surface of each upper punch 82 ··· are discharged in the suction port 95h by the substantial laminar air flow (negative flow). As a result, lubricant (powder) L is homogeneously applied on the surface (lower surface) of each upper punch 82 ··· in just proportion.

Granulated material stored in the storage hopper (not shown) is sequentially charged in the space formed by each die 84 ··· on which surface (inner circumference) the lubricant (powder) L is homogeneously sprayed, and on each lower punch 83 ··· on which surface (upper surface) the lubricant (powder) L is homogeneously sprayed and which is inserted in each die 84 ··· into a fixed position from the molding material charge means (feed chute) 121.

Next, the extra amount of molding material $\underline{m}$ supplied in the space formed by the die 84 and the lower punch 83, inserted in the die 84, is scraped and removed by the scraper 122 provided at the end of the molding material charge means (feed chute) 121.

According to the above-mentioned processes, molding material $\underline{m}$ supplied in the space formed by the surface (inner circumference) of the die 84 and the surface (upper surface) of the lower punch 83 which is inserted in a fixed position in the die 84, is pre-compressed by the upper punch 82 on which surface (lower surface) the lubricant (powder) L is homogeneously applied and by the lower punch 83 on which surface (upper surface) the lubricant (powder) L is homogeneously applied. Then the material m is further compressed at the actual compression point P4 to form a tablet t. Thus, the produced tablet t is discharged at a fixed place by the tablet discharge arm 131 at the tablet discharge position P5.

In this embodiment each one of the numerous upper punches 82 ···, each one of the numerous lower punches 83 ···, each one of the numerous dies 84 is fed to the cleaning position P6 after the tablet t is discharged at the tablet take out position P5. Remaining lubricant (powder) L and/or molding material m (granulated material in this embodiment) are completely removed by each of upper punch cleaning means (not shown), lower punch cleaning means (not shown), and die cleaning means (not shown) provided at the cleaning position P6 for preparing the next tabletting procedure.

According to the external lubricant spraying type tabletting machine 51, the lubricant (powder) L mixed and dispersed in the pulsating vibration air is sprayed in a short time on each surface (upper surface) of the lower punches 83 ··· on which extra lubricant is easily attached because of gravity. Further, the lubricant (powder) L is also applied on each surface (inner circumference) of the die 84 ··· in a short time by the pulsating vibration air. Further the lubricant is homogeneously attached on the surface (upper surface) of the lower punch 83 ··· and the surface (circumferential wall) of the die 84 ···by sucking and removing the extra lubricant (powder) L.

The lubricant (powder) L is applied on each surface (lower surface) of the upper punch 82 ···, on which lubricant has difficulty attaching, in a different way from the method of applying lubricant on the surface (upper surface) of the lower punch 83 ··· and the surface (inner circumference) of the die 84 ···. Namely, the lubricant (powder) L is homogeneously attached on each surface (lower surface) of the upper punch 82 ··· passing in the upper punch containing groove g2 under substantial laminar air flow (negative flow) for a long time while the upper punch 82 moves from the tip es to the end ee of the slit 93s in the upper punch lubrication means 93.

According to the external lubricant spraying type tabletting machine 51, the lubricant (powder) L is applied on each surface (upper surface) of the lower punch 83 ··· and each surface (inner circumference) of the die 84 ··· under positive pressure, such that positive pulsating vibration air mixed with the lubricant (powder) L is sprayed under turbulent airflow and in a short time. On the other hand, the lubricant (powder) L is applied on each surface (lower surface) of upper punch 82 ··· under negative pressure, under substantial laminar air flow and for a long time. Thus, the lubricant (powder) L is applied on each surface (upper surface) of the lower punch 83 ···, each surface (inner circumference) of the die 84 ···, and each surface (lower surface) of the upper punch 82 ··· by suitable methods.

Thus, the lubricant (powder) L can be homogeneously applied on each surface (lower surface) of the upper punch 82 ···, each surface (upper surface) of the lower punch 83 ···, and each surface (inner circumference) of the die 84 ···. Therefore, when a tablet is tabletted without including lubricant, grinding is not caused for the upper punch 82 ···, the lower punch 83 ··· and the die 84 ···, and the molding material m (granulated material in this embodiment) does not attach on them so that tabletting problems such as sticking do not happened for the produced tablet t.

The tablet tabletted without including lubricant therein as shown in this embodiment, can be produced by the several methods shown in the prior art of the present specification. However, when such an external lubricant spraying type tabletting machine 51 is used, lubricant can be homogeneously applied on each surface (upper surface) of the lower punch 83 ···, each surface (inner circumference) of the die 84 ···, and each surface (lower surface) of the upper punch 82 ···, compared with those prior methods so that the tablet of the present invention can be produced with a high productive efficiency.

Further according to the external lubricant spaying type tabletting machine 51, the light scattering type powder density measuring means 112 is provided for the conduit C2 for measuring the density of the lubricant (powder) L flowing in the conduit C2. Therefore, the density of the lubricant (powder) L sprayed from the lubricant spraying means 91 can be controlled also by adjusting the driving amount of the suction means 101 based on the detected value of the light scattering type powder density measuring means 112.

Still further, according to the external lubricant spaying type tabletting machine 51, the lower punch lubrication means 92 of the lubricant spraying means 91 has a long suction port h2 provided on the lower surface S92b of the lower punch lubrication means 92 so as to direct from the periphery to the center of the rotary table 85.

If such a lubricant spraying means 91 having the suction port h2 is used, trial tabletting is executed before executing full tabletting, then the produced sample tablet is pulverized and the amount of lubricant L contained in one tablet is measured. If the amount of lubricant L contained in one tablet is larger than the predetermined amount, a part of the lubricant (powder) L attached on each surface (inner circumference) of the die 84 ··· is sucked and removed or a part of the lubricant (powder) L attached on each surface (upper surface) of the lower punch 83 ··· is sucked and removed by driving the suction means (not shown) connected to the suction port h2 at a proper driving amount.

If the lubricant (powder) L is attached around each die 84 ⋯ of the rotary table 85 or there is such a fear, an operator may operate the suction means (not shown) connected to the suction port h2 at appropriate times or full-time, at proper driving amount so as to clean around each die 84 ⋯ of the rotary table 85. When the suction means (not shown) connected to the suction port h2 is operated in such a manner, tablet without including lubricant can be produced so that an intrabuccally rapidly disintegrable tablet, which is more rapidly dissolved in the mouth, can be produced. The disintegration time in the mouth can differ from one intrabuccally rapidly disintegrable tablet to the next, by changing the combination ratio of saccharide with high wettability against water included in binder so that the present invention has an effect of controlling disintegration time in the mouth.

Industrial Applicability

As explained above, according to the tablet of the present invention, a saccharide with high wettability against water is used and the particle of granulated material is bound by a binder including a saccharide with high wettability against water. According to this construction, when the tablet is given in the mouth, the saccharide with high wettability against water in the binder rapidly gets moistened by the saliva and dissolves or disperses therein. Therefore, the binding force of the binder particle becomes weak and the granulated material is dissolved so that the tablet is rapidly dissolved.

Since the particles comprised in the tablet are bound by a binder and that the binding force is strong, the tablet does not get chipped during storage and transportation.

Furthermore, in specific tablets of the present invention, a functional coating (for example an enteric coating) may be applied on granules including the principal agent so that the principal agent dissolves at a targeted location, a sustained release coating may be applied so that the tablet dissolves gradually, a solid dispersing granule may be prepared so as to prevent the crystallization of the principal agent, and a principal agent may be made with granules dispersed in a wax matrix construction so as to achieve an intrabuccally rapidly disintegrable pharmaceutical formulation.

The tablet described in claim 2 further includes the saccharide particle with high moldability in the granulated material so that such tablet's moldability is even higher than that of the tablet described in claim 1.

In the tablet described in claim 3, the blend ratio of the particles of wettable saccharide and the particles of saccharide with high moldability is set so as to produce a tablet having an excellent moldability of compression which may rapidly disintegrate in the mouth. Therefore, a high productivity of tablets which may rapidly disintegrate in the mouth can be achieved.

According to the tablet described in claim 4, a saccharide which is excellent in safety and moldability and is available is selected as a saccharide with high moldability so that a tablet with high safety, moldability and rapid disintegrability in the mouth can be easily produced.

According to the tablet described in claim 5, a saccharide which is excellent in safety and wettability and is easily available is selected as a saccharide with high wettability against water, so that a tablet which has excellent safety and which can be rapidly disintegrated in the mouth can be easily produced.

Furthermore, because the viscosity of the solution of the saccharide with high wettability against water is not increased when being dissolved in water, the water in the saliva easily permeates the tablet. The tablet is thereby rapidly dissolved by the saliva in the mouth.

In the tablet described in claim 6, the particles are bound by a binder including a surface active agent other than a saccharide with high wettability against water.

Therefore, the binder becomes easily wettable because the surface tension of water in saliva is lowered by a surface active agent in the binder when the tablet is in the mouth. The saccharide with high wettability against water from the binder is quickly wetted and dissolved or dispersed in the saliva. Accordingly, the binding force of the binder is lost and the granulated material is dissolved rapidly.

In the tablet described in claim 7, the particle of granulated material is bound by a water-soluble polymer and a saccharide with high wettability against water. Therefore, a binder is permeated in water from the saliva in the mouth. As a result, because the granulated material is quickly disintegrated and dispersed into particle size, the tablet can be rapidly disintegrated in the mouth.

Furthermore, according to the tablet, the particles of saccharide with high wettability against water are dispersed in a water-soluble polymer binding the particles comprised in the granulated material. According to such a tablet, the particles of saccharide with high wettability against water dispersed in a water-soluble polymer are dissolved into the saliva when they contact the saliva in the mouth. According to such a construction, when the tablet is in the mouth, the saccharide with high wettability against water in the binder quickly gets wet by the saliva therein and dissolves or disperses in the saliva. As a result, the binding force of the particles in the binder become weak so that such tablet is rapidly disintegrated compared with a tablet in which the particles comprised in the granule are bound only by a water-soluble polymer.

According to the tablet production method described in claim 8, an intrabuccally rapidly disintegrable tablet can be produced by means of a fluid bed granulation method and a compression mold method used for producing a conventional tablet, so that no new special apparatus is required for producing this tablet.

In the tablet produced by such a production method, the granulated material included therein is bound by a binder including a saccharide with high wettability against water. Therefore, it is superior in disintegrability in the mouth compared with that of a tablet comprising granulated material with a conventional binder.

Also according to the tablet production method described in claim 9, an intrabuccally rapidly disintegrable tablet can be produced by means of a fluid bed granulation method and a compression mold method used for producing a conventional tablet so that no new and special apparatus is required for producing this tablet.

In the tablet produced by such a production method, the particles comprised in the granulated material included in the tablet are bound by a binder including a saccharide with high wettability against water. Therefore, it is superior in disintegrability in the mouth compared with a tablet in which the granulated material is bound with a binder which includes only a water-soluble polymer is compressed.

Furthermore, according to this production method, the particles of saccharide with high moldability are included in the granulated material. Thereby, tabletting problems such as sticking are avoided.

According to the tablet production method described in claim 10, a surface active agent is added in the binder. Therefore, in the tablet produced by this method, the particles comprised in the granulated material included in the tablet, are bound by a binder including a surface active agent other than a saccharide with high wettability against water, so that such the tablet is more rapidly disintegrated in the mouth.

According to the tablet production method described in claim 11, a water-soluble polymer is used as a binder and same is permeated with water of the saliva when the tablet produced by this production method touches the saliva in the mouth. Therefore, the granulated material is quickly dissolved and dispersed into particle level and rapidly disintegrated in the mouth.

Further according to such a tablet, the particles of saccharide with high wettability against water are dispersed in a water-soluble polymer binding the particles comprised in the granulated material. Therefore, when such a tablet produced by this production method touches saliva in the mouth, the particles of saccharide with high wettability against water are dispersed and the water-soluble polymer is dissolved with the saliva. According to such construction, when the tablet is inserted in the mouth, the saccharide with high wettability against water in the binder quickly gets wet and dissolves or disperses in the saliva. Therefore, the binding force of the particles in the binder becomes weak and the granulated material is disintegrated so that the tablet is rapidly disintegrated.

According to the tablet production method described in claim 12, the ratio of the binder and the saccharide with high wettability against water included in the aqueous solution used for the granulation is adjusted in such a manner that the compressed tablet has an appropriate hardness and the tablet is rapidly dissolved in the mouth. Therefore, an intrabuccally rapidly disintegrable tablet which hardly chips during storage and transportation and which is rapidly dissolved in the mouth can be produced.

**Claims**

1. An intrabuccally rapidly disintegrating tablet produced by granulating a powdered mixture comprising a principal agent, a saccharide with high wettability against water, and a disintegrant using an aqueous solution comprising a binder and a saccharide with high wettability against water to make a granulated material, drying the granulated material, and compressing the dried granulated material,
   wherein said binder is a water-soluble polymer and said disintegrant is selected from the group consisting of crosspovidone, crosscarmellose sodium, and low substituted hydroxypropylcellulose.

2. An intrabuccally rapidly disintegrating tablet produced by granulating a powdered mixture comprising a principal agent, a saccharide with high wettability against water, a saccharide with high moldability and a disintegrant using an aqueous solution comprising a binder and a saccharide with high wettability against water to make a granulated material, drying the granulated material, and compressing the dried granulated material,
   wherein said binder is a water-soluble polymer and said disintegrant is selected from the group consisting of crosspovidone, crosscaimellose sodium, and low substituted hydroxypropylcellulose.

3. The tablet as set forth in claim 2, wherein the volume ratio in said granulated material of said saccharide with high wettability against water to said saccharide with high moldability is within the range of 6:4 to 9:1.

4. The tablet as set forth in claim 2 or 3, wherein said saccharide with high moldability is selected from the group consisting of lactose, maltitol, sorbitol, and oligosaccharide.

5. The tablet as set forth in any one of claims 1 to 4, wherein said saccharide with high wettability against water is selected from the group consisting of trehalose, mannitol, maltose, sorbitol, lactose, multitol, xylitol, sucrose, erythritol, and glucose.

6. The tablet as set forth in any one of claims 1 to 4, wherein said saccharide with high wettability against water is selected froan the group consisting of mannitol, maltose and lactose.

7. The tablet as set forth in any one of claims 1 to 6, wherein said aqueous solution further contains a surface active agent.

8. The tablet as set forth in any one of claims 1 to 7, wherein said binder is selected from the group consisting of polyvinylpyrrolidone, hydroxypropylmethylcellulose, partially saponified polyvinyl alcohol, methylcellulose, pullulane, polyvinyl alcohol, and hydroxypropylcellulose.

9. The tablet as set forth in any one of claims 1 to 7, wherein said binder is selected fromthe group consisting of hydroxypropylcellulose and polyvinyl alcohol.

10. A method of producing an intrabuccally rapidity disintegrating tablet, comprising the steps of:

    fluidizing with air a powdered mixture comprising a principal agent, a saccharide with high wettability against water and a disintegrant, producing a granulated material by spraying an aqueous solution comprising a binder and a saccharide with high wettability against water into said powdered mixture and drying said granulated material, and compressing said granulated material to tabletted,

    wherein said binder is a water-soluble polymer and said disintegrant is selected from the group consisting of crosspovidone, crosscarmellose sodium, and low substituted hydroxypropylcellulose.

11. A method of producing an intrabuccally rapidly disintegrating tablet, comprising the steps of:

    fluidizing with air a powdered mixture comprising a principal agent; a saccharide with high wettability against water, a saccharide with high moldability, and a disintegrant, producing a granulated material by spraying an aqueous solution comprising a binder and a saccharide with high wettability against water into said powdered mixture and drying said granulated material, and compressing said granulated material to be tabletted,

    wherein said binder is a water-soluble polymer and said disintegrant is selected from the group consisting of crosspovidone, crossoarmellose sodium, and low substituted hydroxypropylcellulose.

12. The method as set forth in claim 11, wherein the volume ratio in said granulated materials of said saccharide with high wettability against water to said saccharide with high moldability is within the range of 6:4 to 9:1.

13. The method as set forth in claim 11 or 12, wherein said saccharide with high moldability is selected from the group consisting of lactose, maltitol, sorbitol, and oligosaccharide.

14. The method as set forth in any one of claims 10 to 13, wherein said saccharide with high wettability against water is selected from the group consisting of trehalose, mannitol, maltose, sorbitol, lactose, multitol, xylitol, sucrose, erythritol, and glucose.

15. The method as set forth in any one of claims 10 to 13, wherein said saccharide with high wettability against water is selected from the group consisting of mannitol, maltose and lactose.

16. The method as set forth in any one of claims 10 to 15, wherein said aqueous solution further contains a surface active agents.

17. The method as set forth in any one of claims 10 to 16, wherein said binder is selected from the group consisting of polyvinylpyrrolidone, hydroxypropylmethylcellulose, partially saponified polyvinyl alcohol, methylcellulose, pullulane, polyvinyl alcohol, and hydroxypropylcellulose.

18. The method as set forth in any one of claims 10 to 16, wherein said binder is selected from the group consisting of hydroxypropylcellulose and polyvinyl alcohol.

**Patentansprüche**

1. Eine im Mund schnell zerfallende Tablette, hergestellt durch Granulieren einer pulvrigen Mischung, die einen Wirkstoff, ein Saccharid mit hoher Benetzbarkeit gegenüber Wasser und ein Auflösungsmittel umfasst, unter Verwendung einer wässrigen Lösung, die ein Bindemittel und ein Saccharid mit hoher Benetzbarkeit gegenüber Wasser beinhaltet, um ein granuliertes Material herzustellen, Trocknen des granulierten Materials und Pressen des getrockneten granulierten Materials, wobei das Bindemittel ein wasserlösliches Polymer ist und das Auflösungsmittel ausgewählt ist aus der Gruppe bestehend aus Crosspovidon, Crosscarmelose-Natrium und niedrig substituierter Hydroxypropylcellulose.

2. Eine im Mund schnell zerfallende Tablette, hergestellt durch Granulieren einer pulvrigen Mischung, die einen Wirkstoff, ein Saccharid mit hoher Benetzbarkeit gegenüber Wasser, ein Saccharid mit großer Formbarkeit und ein Auflösungsmittel umfasst, unter Verwendung einer wässrigen Lösung, die ein Bindemittel und ein Saccharid mit hoher Benetzbarkeit gegenüber Wasser beinhaltet, um ein granuliertes Material herzustellen, Trocknen des granulierten Materials und Pressen des getrockneten granulierten Materials, wobei das Bindemittel ein wasserlösliches Polymer ist und das Auflösungsmittel ausgewählt ist aus der Gruppe bestehend aus Crosspovidon, Crosscarmelose-Natrium und niedrig substituierter Hydroxypropylcellulose.

3. Tablette nach Anspruch 2, wobei das Volumenverhältnis des Saccharids mit der hohen Benetzbarkeit gegenüber Wasser zu dem Saccharid mit der großen Formbarkeit in dem granulierten Material im Bereich von 6:4 bis 9:1 ist.

4. Tablette nach Ansprüchen 2 oder 3, wobei das Saccharid mit der großen Formbarkeit aus der Gruppe bestehend aus Lactose, Maltit, Sorbit, und Oligosaccharid ausgewählt ist.

5. Tablette nach einem der Ansprüche 1 bis 4, wobei das Saccharid mit der hohen Benetzbarkeit gegenüber Wasser aus der Gruppe bestehend aus Trehalose, Mannit, Maltose, Sorbit, Lactose, Multit, Xylit, Saccharose, Erythrit und Glucose ausgewählt ist.

6. Tablette nach einem der Ansprüche 1 bis 4, wobei das Saccharid mit der hohen Benetzbarkeit gegenüber Wasser aus der Gruppe bestehend aus Mannit, Maltose und Lactose ausgewählt ist.

7. Tablette nach einem der Ansprüche 1 bis 6, wobei die wässrige Lösung zusätzlich eine oberflächenaktive Substanz enthält.

8. Tablette nach einem der Ansprüche 1 bis 7, wobei das Bindemittel aus der Gruppe bestehend aus Polyvinylpyrrolidon, Hydroxypropylmethylcellulose, teilweise verseiftem Polyvinylalkohol, Methylcellulose, Pullulan, Polyvinylalkohol und Hydroxypropylcellulose ausgewählt ist.

9. Tablette nach einem der Ansprüche 1 bis 7, wobei das Bindemittel aus der Gruppe bestehend aus Hydroxypropylcellulose und Polyvinylalkohol ausgewählt ist.

10. Verfahren zur Herstellung einer im Mund schnell zerfallenden Tablette, umfassend die folgenden Schritte:

Fluidisieren einer pulvrigen Mischung mit Luft, wobei die Mischung einen Wirkstoff, ein Saccharid mit hoher Benetzbarkeit gegenüber Wasser und ein Auflösungsmittel beinhaltet, Herstellen eines granulierten Materials durch Sprühen einer wässrigen Lösung, die ein Bindemittel und ein Saccharid mit hoher Benetzbarkeit gegenüber Wasser beinhaltet, in die pulvrige Mischung und Trocknen des granulierten Materials und Pressen des granulierten Materials zu Tabletten, wobei das Bindemittel ein wasserlösliches Polymer ist und das Auflösungsmittel aus der Gruppe bestehend aus Crosspovidon, Crosscarmelose-Natrium und niedrig substituierter Hydroxypropylcellulose ausgewählt ist.

11. Verfahren zur Herstellung einer im Mund schnell zerfallenden Tablette, umfassend die folgenden Schritte:

Fluidisieren einer pulvrigen Mischung mit Luft, wobei die Mischung einen Wirkstoff, ein Saccharid mit hoher Benetzbarkeit gegenüber Wasser, ein Saccharid mit großer Formbarkeit und ein Auflösungsmittel beinhaltet, Herstellen eines granulierten Materials durch Sprühen einer wässrigen Lösung, die ein Bindemittel und ein Saccharid mit hoher Benetzbarkeit gegenüber Wasser beinhaltet, in die pulvrige Mischung und Trocknen des granulierten Materials und Pressen des granulierten Materials zu Tabletten, wobei das Bindemittel ein wasserlösliches Polymer ist und das Auflösungsmittel aus der Gruppe bestehend aus Crosspovidon, Crosscarmelose-Natrium und niedrig substituierter Hydroxylpropylcellulose ausgewählt ist.

12. Verfahren nach Anspruch 11, wobei das Volumenverhältnis des Saccharids mit der hohen Benetzbarkeit gegenüber Wasser zu dem Saccharid mit der großen Formbarkeit in dem granulierten Material im Bereich von 6:4 bis 9:1 ist.

13. Verfahren nach Anspruch 11 oder 12, wobei das Saccharid mit der großen Formbarkeit aus der Gruppe bestehend aus Lactose, Maltit, Sorbit und Oligosaccharid ausgewählt ist.

14. Verfahren nach einem der Ansprüche 10 bis 13, wobei das Saccharid mit der hohen Benetzbarkeit gegenüber Wasser aus der Gruppe bestehend aus Trehalose, Mannit, Maltose, Sorbit, Lactose, Multit, Xylit, Saccharose, Erythrit und Glucose ausgewählt ist.

15. Verfahren nach einem der Ansprüche 10 bis 13, wobei das Saccharid mit der hohen Benetzbarkeit gegenüber Wasser aus der Gruppe bestehend aus Mannit, Maltose und Lactose ausgewählt ist.

16. Verfahren nach einem der Ansprüche 10 bis 15, wobei die wässrige Lösung zusätzlich eine oberflächenaktive Substanz enthält.

**17.** Verfahren nach einem der Ansprüche 10 bis 16, wobei das Bindemittel aus der Gruppe bestehend aus Polyvinylpyrrolidon, Hydroxypropylmethylcellulose, teilweise verseiftem Polyvinylalkohol, Methylcellulose, Pullulan, Polyvinylalkohol und Hydroxypropylcellulose ausgewählt ist.

**18.** Verfahren nach einem der Ansprüche 10 bis 16, wobei das Bindemittel aus der Gruppe bestehend aus Hydroxypropylcellulose und Polyvinylalkohol ausgewählt ist.

**Revendications**

**1.** Comprimé à désintégration intra-buccale rapide produit par granulation d'un mélange sous forme de poudre comprenant un agent principal, un saccharide ayant un taux de mouillabilité contre l'eau élevé, et un désintégrant, en utilisant une solution aqueuse comprenant un liant et un saccharide ayant un taux de mouillabilité contre l'eau élevé pour produire un matériau granulé, séchage du matériau granulé, et compression du matériau granulé séché, dans lequel ledit liant est un polymère soluble dans l'eau et ledit désintégrant est choisi dans le groupe constitué de crosspovidone, crosscarmelose sodium et d'hydroxypropylcellulose faiblement substituée.

**2.** Comprimé à désintégration intra-buccale rapide produit par granulation d'un mélange sous forme de poudre comprenant un agent principal, un saccharide ayant un taux de mouillabilité contre l'eau élevé, un saccharide ayant une aptitude au moulage élevée et un désintégrant, en utilisant une solution aqueuse comprenant un liant et un saccharide ayant un taux de mouillabilité contre l'eau élevé pour produire un matériau granulé, séchage du matériau granulé, et compression du matériau granulé séché, dans lequel ledit liant est un polymère soluble dans l'eau et ledit désintégrant est choisi dans le groupe constitué de crosspovidone, crosscarmelose sodium et d'hydroxypropylcellulose faiblement substituée.

**3.** Comprimé selon la revendication 2, dans lequel le rapport volumique dans ledit matériau granulé du saccharide ayant un taux de mouillabilité contre l'eau élevé sur le saccharide ayant une aptitude au moulage élevée est dans la zone de 6 :4 à 9 :1.

**4.** Comprimé selon la revendication 2 ou 3, dans lequel ledit saccharide ayant une aptitude au moulage élevée est choisi dans le groupe constitué de lactose, maltitol, sorbitol et d'oligosaccharide.

**5.** Comprimé selon l'une quelconque des revendications 1 à 4, dans lequel ledit saccharide ayant un taux de mouillabilité contre l'eau élevé est choisi dans le groupe constitué de tréhalose, mannitol, maltose, sorbitol, lactose, multitol, xylitol, sucrose, érythrite et glucose.

**6.** Comprimé selon l'une quelconque des revendications 1 à 4, dans lequel ledit saccharide ayant un taux de mouillabilité contre l'eau élevé est choisi dans le groupe constitué de mannitol, maltose et lactose.

**7.** Comprimé selon l'une quelconque des revendications 1 à 6, dans lequel ladite solution aqueuse contient en outre un agent de surface actif.

**8.** Comprimé selon l'une quelconque des revendications 1 à 7, dans lequel ledit liant est choisi dans le groupe constitué de polyvinylpyrrolidone, hydroxypropylméthylcellulose, un alcool polyvinyl partiellement saponifié, la méthylcellulose, le pullulane, un alcool polyvinyl et l'hydroxypropylcellulose.

**9.** Comprimé selon l'une quelconque des revendications 1 à 7, dans lequel ledit liant est choisi dans le groupe constitué d'hydroxypropylcellulose et d'un alcool polyvinyl.

**10.** Procédé de production d'un comprimé à désintégration intra-buccale rapide, comprenant les étapes consistant à :

    fluidiser par l'air un mélange sous forme de poudre comprenant un agent principal, un saccharide ayant un taux de mouillabilité contre l'eau élevé et un désintégrant, produire un matériau granulé par pulvérisation, dans ce mélange sous forme de poudre, d'une une solution aqueuse comprenant un liant et un saccharide ayant un taux de mouillabilité contre l'eau élevé, et sécher ledit matériau granulé, et comprimer ledit matériau granulé pour former un comprimé, dans lequel ledit liant est un polymère soluble dans l'eau et ledit désintégrant est choisi dans le groupe constitué de crosspovidone, crosscarmelose sodium et d'hydroxypropylcellulose faiblement substituée.

**11.** Procédé de production d'un comprimé à désintégration intra-buccale rapide, comprenant les étapes consistant à :

    fluidiser par l'air un mélange sous forme de poudre comprenant un agent principal, un saccharide ayant un taux de mouillabilité contre l'eau élevé, un saccharide ayant une aptitude au moulage élevée et un désintégrant, produire un matériau granulé par pulvérisation, dans ce mélange sous forme de poudre, d'une une solution aqueuse comprenant un liant et un saccharide ayant un taux de mouillabilité contre l'eau élevé, et sécher ledit matériau granulé, et comprimer ledit matériau granulé pour former un comprimé, dans lequel ledit liant est un polymère soluble dans l'eau et ledit désintégrant est choisi dans le groupe constitué de crosspovidone, crosscarmelose sodium et d'hydroxypropylcellulose faiblement substituée.

12. Procédé selon la revendication 11, dans lequel le rapport volumique dans ledit matériau granulé du saccharide ayant un taux de mouillabilité contre l'eau élevé sur le saccharide ayant une aptitude au moulage élevée est dans la zone de 6 :4 à 9 :1.

13. Procédé selon la revendication 11 ou 12, dans lequel ledit saccharide ayant une aptitude au moulage élevée est choisi dans le groupe constitué de lactose, maltitol, sorbitol et d'oligosaccharide.

14. Procédé selon l'une quelconque des revendications 10 à 13, dans lequel ledit saccharide ayant un taux de mouillabilité contre l'eau élevé est choisi dans le groupe constitué de tréhalose, mannitol, maltose, sorbitol, lactose, multitol, xylitol, sucrose, érythrite et glucose.

15. Procédé selon l'une quelconque des revendications 10 à 13, dans lequel ledit saccharide ayant un taux de mouillabilité contre l'eau élevé est choisi dans le groupe constitué de mannitol, maltose et lactose.

16. Procédé selon l'une quelconque des revendications 10 à 15, dans lequel ladite solution aqueuse contient en outre un agent de surface actif.

17. Procédé selon l'une quelconque des revendications 10 à 16, dans lequel ledit liant est choisi dans le groupe constitué de polyvinylpyrrolidone, hydroxypropylméthylcellulose, un alcool polyvinyl partiellement saponifié, la méthylcellulose, le pullulane, un alcool polyvinyl et l'hydroxypropylcellulose.

18. Procédé selon l'une quelconque des revendications 10 à 16, dans lequel ledit liant est choisi dans le groupe constitué d'hydroxypropylcellulose et d'un alcool polyvinyl.

*Fig.1*

(a)

(b)

| water | water-soluble polymer | saccharide with high wettability against water |

water solution

(c)

Fig.2

(a)

(b)

*Fig.3*

(a)

(b)

(c)

(d)

*Fig.4*

*Fig.5*

(a)

(b)

| water | water-soluble polymer | saccharide with high wettability against water |

water solution

(c)

Fig.6

(a)

(b)

*Fig.7*

(a)

Tb

R3

(b)

4

9

3

R4

6

9

(c)

3

7
8} 6

4

2

(d)

3

7

4

8

2

*Fig.8*

*Fig.9*

(a)

(b)

| water | water-soluble polymer | saccharide with high wettability against water |

water solution ← surface active agent

(c)

*Fig.10*

(a)

(b)

*Fig.11*

(a)

Tc

R5

(b)

3

2

R6

4

6c

(c)

3

6c { 8
7
10

4

2

(d)

3

10

7

8

4

2

*Fig.12*

*Fig.13*

EP 1 161 940 B1

*Fig.14*

68h

R

C1

68b

63

62

64

68

69

air source

flow rate controller

V

C3

68c

68a

70h

70

69a

66

69b

p1

p2

65a

67b

67

67a

*Fig.15*

*Fig.16*

73

73s

*Fig.17*

( a )

( b )

( c )

*Fig.18*

*Fig.19*

rotational direction of rotary table

*Fig.20*

*Fig.21*

rotational direction of rotary table

*Fig.22*